# EUROPEAN PATENT APPLICATION

(11) **EP 2 034 010 A1**
(43) Date of publication of application: **11.03.2009**
(21) Application number: 07115352.2
(22) Date of filing: 30.08.2007
(51) Int. Cl.: C12N 5/06, A61L 27/22, A61L 27/50

(54) **Compositions suitable for repair and/or treatment of injured spinal tissue**

(71) Applicant: Omrix Biopharmaceuticals Ltd., 76106 Rehovot (IL)
(72) Inventor: Bechor, Edna, Ramat Gan 52255 (IL); Bar, Lily, Rehovot (IL); Nur, Israel, Moshav Timorim (IL)
(74) Representative: Meyers, Hans-Wilhelm

(57) **Abstract**

The invention relates to the use of viral inactivated-plasma cryoprecipitate concentrate (VIPCC) comprising a suitable fibronectin/fibrinogen ratio for treating, regenerating, repopulating injured Nucleus Pulposus cells/tissue, notochord derived tissue, and/or tissue whose formation is guided by notochordal cells, e.g. spinal intervertebral disc and/or central nervous system such as spine.

## Description

### Field of the invention

The invention relates to the use of a viral inactivated-plasma cryoprecipitate concentrate comprising an initial relative concentration of fibrinogen and fibronectin suitable for treatment of tissue injury, repair of injured tissue and/or slow or reversing degeneration of a tissue such as a spinal tissue, like intervertebral disc.

### Background of the invention

Human intervertebral disc degeneration is a clinical problem, and leading cause of spinal pain and disability. Over 15 million people worldwide suffer from disc degeneration, which is typically characterized by having an altered matrix composition and reduced cell number.

Intervertebral discs lie between adjacent vertebrae in the spine. Each disc forms a cartilaginous joint that allows slight movements of the vertebrae, and acts as a ligament that holds the vertebrae together. The intervertebral disc is composed of an outer band called the Annulus Fibrosus (AF) and a gel-like inner substance called the Nucleus Pulposus (NP).

The embryonic development consists of three germ layers: the endoderm, mesoderm, and ectoderm. These layers ultimately give rise to the internal organs, the musculoskeletal tissues, and the epidermal and nervous tissues, respectively. A fourth region known as the notochord guides the embryonic development of the neural tube, and the vertebral column, including the intervertebral discs.

During embryonic development, mesenchymal cells begin to migrate and condense around the notochord to form the vertebrae and Annulus Fibrosus. The entrapped notochordal cells are believed to take part in formation of the Nucleus Pulposus. Thus, the Annulus Fibrosus and the Nucleus Pulposus are originated from different embryonic origin.

The Annulus Fibrosus is primarily composed of type I collagen fibrils that form concentric lamellae that surround the nucleus and insert into the endplates of the adjacent vertebral bodies to form a reinforced structure.

The Nucleus Pulposus tissue consists of predominantly chondrocytes-like cells and larger, highly vacuolated cells which are presumed remnants of the notochord (Hunter et al, 2003). The chondrocytes-like cells express type II collagen, proteoglycan aggrecans, and hyaluronan long chains, which have molecules with highly hydrophilic, branching side chains. These negatively charged regions have a strong avidity for water molecules and hydrate the nucleus or center of the disc by an osmotic swelling pressure. The hydraulic effect of the contained hydrated nucleus within the annulus acts as a shock absorber to cushion the spinal column from forces that are applied to the musculoskeletal system.

Degenerative Disc Disease (DDD) is an undesired process in which the intervertebral discs lose their flexibility, elasticity, and shock absorbing characteristics. The collagen structure of the Annulus Fibrosus weakens and becomes brittle. Excessive pressure on a weakened disc can cause tears in Annulus Fibrosus and the Nucleus Pulposus to squeeze through the tears, causing the disc to rupture (herniated disc) into the space that surrounds a nerve root or the spinal canal. A herniated disc can interfere with nerve function, leading to weakness, numbness, inflammation and pain. Additionally, proteoglycan content decreases, thereby reducing the water retaining capabilities of the Nucleus Pulposus. These changes reduce the ability of the discs to act as shock absorbers and make them less flexible. Loss of fluid also makes the disc smaller and narrows the distance between the vertebrae and the disc.

Since the intervertebral discs are located in a non-vascular environment, they have a limited capacity for repair. If intervertebral disc can be rejuvenated, the degenerative process could slow or reverse.

Degeneration of intervertebral disc, or a hematoma can cause spinal cord compression and spinal injury.

Spinal Cord Injury (SCI) - spinal cord injury usually begins with trauma to the spine that damages the nerves within the spinal canal. Frequent causes of damage are trauma (car accident, gunshot, falls, etc.) or disease (polio, spina bifida, Friedreich's Ataxia, etc.) and compression of the spine by intervertebral disc herniation. An injury to the nerves results in loss or deficit in motor, sensory, and autonomic function. Secondary injury following the primary impact includes a number of biochemical and cellular alterations leading to tissue necrosis and cell death. It is estimated that the annual incidence of SCI is approximately 40 cases per million in the U. S., and that the cost of managing the care of SCI patients approaches $4 billion each year. To date, relatively little progress has been made in the treatment of SCI and related neurological impairments. Currently there is only one accepted, although unapproved, therapy, methylprednisolone. If used in very high doses no later than 8 hours after the injury, methlyprednisolone has demonstrated a modest ability to improve the neurological outcome following SCI.

In the developing vertebrate nervous system, the neural tube is the precursor of the central nervous system (CNS), which comprises the brain and spinal cord. The spinal cord contains communication fibers called axons that transfer sensory and motor information between the brain and the periphery. In transverse section the spinal cord is divided into symmetrical halves by a dorsal median and a ventral median. The dorsal part of the neural tube is primarily associated with sensation, whereas the ventral part is primarily associated with motor (e,g. muscle) control. The term motor neuron classically applies to neurons located in the CNS which project their axons outside the CNS and directly or indirectly control muscles. The term is synonymous with efferent neurons.

Research shows that by transplanting and expressing a second notochord near the dorsal neural tube, 180 degrees opposite of the normal notochord location, one can induce the formation of motor neurons in the dorsal tube, which generally forms sensory cells. Therefore, it is conceivable that transplantation of Nucleus Pulposus along an injured spinal cord might stimulate the growth of damaged axons. An injured CNS is a highly inhibitory environment for axon regeneration, severely limiting functional recovery following injury. Thus, allocating a specific tissue scaffold that can maintain Nucleus Pulposus cells and progenitors thereof play a key role in the regeneration and functional recovery of spinal core or other Central Nerve System injuries.

Human Fibrin glue is a blood product obtained from either commercial sources of some regional blood transfusion centers. Valuable components for the preparation of the fibrin glue are fibrinogen, thrombin Factor VIII, Factor XIII, fibronectin, vitronectin, von Willebrand factor (vWF), etc. Some manufacturers add anti-proteolytic agents (as described in WO-A-93/05822) or specifically remove the plasminogen in order to delay the fibrinolytic action of plasmin (as described in US Patent 5,792,835 and US Patent 7,125,569).

Bar et al (2005) reported that preparation of fibrin gel formulations prepared from cryoprecipitate differ in their content of fibronectin and heteronectin (fibrinogen-fibronectin covalently linked complexes). The content of these complexes in the formulation influence the adhesion of the fibrin based adhesive to collagen, a structural protein of the extracellular matrix. Cryoprecipitation from plasma is the first step for manufacture of fibrinogen for the fibrin based adhesive. On the one hand, Schwartz et al (US patent 4,377,572) purification procedure results in removal of most of the cross linked fibrinogen-fibronectin molecules and collagen and gelatin-binding fraction. The ratio of fibronectin:fibrinogen in the formulation is of 1/14.7. On the other hand, the cryoprecipitate preparation described by Martinowitz and

Bal (EP patent 691,858) preserves these cross linked fibrinogen-fibronectin molecules, and consequently have an increased fibronectin/fibrinogen ratio of 1/7 which correlates with increased adherence to collagen as compared to adherence to collagen of the preparation obtained by the Schwartz purification.

Three-dimensional fibrin matrices have been used as cellular substrates in vitro and as bridging materials for central nervous system repair. Ju et al (2007) reported that salmon fibrin gels were superior scaffold for neuronal re-growth after CNS injury than fibrin prepared from human or bovine blood proteins.

Cheng et al (1996) repaired spinal cord gaps in adult rats using peripheral nerve grafts. The grafted area was stabilized with fibrin glue containing acidic fibroblast growth factor.

US patent application 2004/0121011 describes a method for promoting repair, regeneration and re-growth of injured neuronal cells. The formulation combines Rho antagonist and a flowable carrier component capable of forming an acceptable matrix in vivo such as tissue adhesives. The application discloses different protein-based tissue adhesives including collagen gels, fibrin tissue adhesives, matrigel etc. Various commercial preparations are disclosed such as, Tissucol® / TISSEEL^{®}, Beriplast® P, and

Disc cells grown in monolayer assume a fibroblast-like phenotype. In a three-dimensional environment, however, disc cells become rounded, form colonies, and exhibit greater proliferation and proteoglycan synthesis. Various in vitro culture techniques, including complex three-dimensional gels and degradable polymer scaffolds have been developed with the goal of providing a sustainable frame on which the disc cells can proliferate. Hyaluronic acid, collagen, chitosan and fibrin gel have been used in cross-linkable polymeric preparation to entrap cells. Nevertheless, Gruber et al (2004) reported that fibrin gel formulations were inferior microenvironments for proliferation, ECM production and gene expression of Annulus Fibrosus cells.

US patent 6,428,576 describes a method for in-situ curing sealant to treat defects in the Annulus Fibrosus. The patent discloses a formulation that cure to a viscoelastic member that simulates the structure, physical properties and biomechanical functions of the Annulus Fibrosus. The cured polymer may be synthetic or naturally occurring. The patent discloses that synthetic polymers are more reliable. The patent discloses several natural occurring proteins, such as fibrin, protein fractionated from blood or recombinant proteins, including processed, denatured or otherwise modified. The patent does not mention a specific composition of the in-situ curing sealant or a specific purification procedure of the fractionated blood proteins. According to the invention, fibrinogen or fibrin from any source may be used.

W007089942A2 discloses a process for forming a kit, comprising: providing a fibrinogen component, an activating compound, and a fibrin sealant delivery apparatus for injecting fibrin sealant into a human disc and a method of treating a spinal joint, comprising injecting a fibrin sealant into a joint to seal at least one defect of a fibrous joint capsule while monitoring the pressure of the fibrin sealant being injected, wherein the fibrin sealant comprises fibrinogen and an activating compound, wherein the fibrinogen and activating compound forms at least a portion of the fibrin after injection. The description discloses use of any fibrinogen that will form fibrin in a human body and not a particular cryoprecipitate composition. Fibronectin is only mentioned as one out of numerous possible additives.

WO 2006/050268 discloses an injection of fibrin sealant into a tear or a fissure in the Annulus Fibrosus. The sealant comprises fibrinogen and activating compound such as thrombin. According to the application, the fibrinogen component can be autologous, human including pooled human fibrinogen, recombinant, and bovine or other non-human source. Fibronectin is mentioned among many components as an additional additive which can be employed in the fibrin sealant. The description of WO 2006/050268 discloses that alternative amounts of fibrinogen may be used in order to change the density of the combined components, but neither the relative ratio to the fibronectin component nor its minimal concentration is disclosed.

Also WO 2006/050267 discloses an injection of fibrin sealant and anesthetic into the spinal area to treat leaky disc syndrome, such as tears or fissures in the Annulus Fibrosus. According to the invention, the fibrinogen component includes any fibrinogen that will form fibrin in human body. The patent mentions commercial kits from manufacturers as Baxter such as TISSEEL^{®}. The description of WO 2006/050267 discloses that alternative amounts of fibrinogen may be used in order to change the density of the combined components. The patent application does not relate to the ratio of the fibrinogen component relative to fibronectin. The injection may also serve to coat nerve roots and increase the disc space thus relieving pressure on the nerve root.

Tissue engineering is an alternative biologic approach, which aims to replace, repair, maintain, and/or enhance tissue function by combination of cells, suitable biochemical and physiochemical factors and optionally a porous structure to be employed as scaffold.

US patent application 2006/0275273, describes a method for implantation or injection of chondrocytes into a degenerative intervertebral disc. The patent, discloses chondrocytes obtained from cadaver. The description discloses several biomolecules to be added to the composition such as laminin, chitosan, hydrogel, collagen type I, II, III, fibrinogen, fibrin, thrombin, fibronectin etc. Various commercial comprising such molecules can be used, such as, TISSEEL^{®} fibrin glue. The examples also disclose cryoprecipitated porcine fibrinogen and a chondrocyte-thrombin solution. The patent application is silent on the ratio of the components relative to fibrinogen.

US patent application 2007/0093905 discloses a mixture for repair and regeneration of intervertebral discs comprising glycine, concentrated monocytes and fibrin glue. The patent also discloses injection of treated isolated disc cells. The reinserted nucleus or annular tissue can optionally be combined with carriers such as gel-like carrier or adhesive. The mentioned adhesives include hyaluronic acid, collagen gel, mussel-based adhesive, fibrin glue, fibrin clot, blood, blood clot, blood component, blood component clot etc. The patent application does not mention a specific concentration or composition of the cryoprecipitate, purified clotting proteins, recombinant clotting proteins, and is silent on relative amount of the components to the fibrinogen.

There is a need for an optimal composition suitable for treating spine injury, enhancing intravertebral disc repair, and/or slowing or reversing disc degeneration.

### Summary of the invention

Fibrin glues are well known and are used extensively in various clinical setting. Such glues are used in surgery, both as a useful addition to sutures and to provide optimal wound integrity, and for preventing or treating adhesions. Recently, literature on the use of fibrin glue to reconstruct spinal intervertebral disc, sealing fissures in the Annulus Fibrosus and in central nervous system restoration has been reported. Some fibrin glue components, such as cryoprecipitate comprise fibronectin in addition to fibrinogen.

It was found according to the present invention that an increase in fibrinogen concentration in coated plates significantly decreased the attachment of the intervertebral disc Nucleus Pulposus cells to the plates, while an increase in fibronectin concentration lead to the opposite outcome. On the other hand a fibrin glue composition such as cryoprecipitate which is selectively depleted of fibrinogen and comprises high levels of fibronectin would not cure and therefore will not form a scaffold which supports a three-dimensional tissue formation. The present invention solves this technical problem and provides cryoprecipitate with optimal fibronectin and fibrinogen concentrations.

It was found according to the present invention that fibronectin/fibrinogen ratio plays a crucial role in the Nucleus Pulposus cell attachment, proliferation and migration and absolutely depends on a high content of fibronectin relative to fibrinogen.

For example, it was found according to the invention that Nucleus Pulposus cells cultured in a mixture which comprises an increased fibronectin/fibrinogen ratio of about 1/10-1/5 show increased cell-attachment and proliferation as compared to cells cultured in a mixture which comprises a lower fibronectin/fibrinogen ratio. Thus, a cryoprecipitate comprising a fibronectin/fibrinogen ratio higher than 1/12 and in the range of 1/10-1/5, such as 1/7 can be used as a superior component of a matrix for growing Nucleus Pulposus cells.

Studies indicate that notochord cells found in the Nucleus Pulposus tissue can guide the formation of Nucleus Pulposus and CNS cells and/or can be used as progenitors of such cells. Therefore, Nucleus Pulposus cells can be used to assist in reconstruction of the intervertebral disc and the central nervous system (CNS).

In one aspect, the invention provides **a** vehicle or construct suitable for delivering a composition of cells comprising notochordal cells or notochordal derived cells into a damaged tissue comprising viral inactivated-plasma activated cryoprecipitate concentrate (VIPCC) having an initial fibronectin/fibrinogen relative concentration higher than 1/12 or about 1/11 to about 1/5 and notochordal cells and/or notochordal derived cells.

The vehicle according to the invention is for delivering cells to intervertebral disc and/or injured central nervous system such as injured spine.

In an embodiment of the present invention, the composition of cells comprises Nucleus Pulposus cells.

In a further embodiment of the invention, the composition of cells further comprises Annulus Fibrosus cells.

Another aspect of the invention relates to a kit for injecting therapeutic solutions into a damaged spinal intervertebral disc and/or injured central nervous system such as injured spinal cord comprising: a first container comprising viral inactivated-plasma cryoprecipitate concentrate (VIPCC) comprising a fibronectin/fibrinogen ratio of higher than 1/12, or about 1/11 to about 1/5, and a second container comprising an enzyme capable of forming fibrin when it reacts with fibrinogen.

The kit comprises at least one container comprising a contrast agent. A non limiting example of a contrast agent in the kit is iodine

One object of the present invention is to provide a kit comprising: a first container comprising a viral inactivated-plasma cryoprecipitate concentrate (VIPCC) having a fibronectin/fibrinogen ratio of higher than 1/12 or about 1/11 to about 1/5, a second container comprising an enzyme capable of forming fibrin when it reacts with fibrinogen, and a third container comprising a proteolytic enzyme selected from serine peptidases, cystein peptidases, aspartic peptidases, metallo peptidases, hyaluronidase and combinations thereof.

The proteolytic enzyme in the kit of the inventioncan can be trypsin, chymotrypsin, pancreatic elastase, papain chymopapain, pepsin, collagenase, gelatinase, pronase chondroitinase, hyaluronidase and combinations thereof.

The kit according to the invention comprises at least one container comprising a contrast agent. In one embodiment of the invention, the contrast agent is iodine.

Another object of the invention is to provide a scaffold suitable for repair and/or regeneration of spinal intervertebral disc and/or injured central nervous system such as injured spinal cord when administered in the body comprising viral inactivated-plasma cryoprecipitate concentrate (VIPCC) having an initial fibronectin/fibrinogen relative concentration of higher than1/12 or about 1/11 to about 1/5.

In an embodiment of the invention, the VIPCC is activated VIPCC.

In another aspect of the invention, the invention provides a tissue or cell bank comprising notochordal cells and/or notochordal derived cells in a scaffold comprising viral inactivated-plasma cryoprecipitate concentrate (VIPCC) having an initial fibronectin/fibrinogen relative concentration of higher than 1/12 or about 1/11 to about 1/5.

In one embodiment of the invention, the cells are Nucleus Pulposus cells.

Another aspect of the invention relates to a method for enhancing growth, proliferation, differentiation, maintenance, repair and/or restoration of a population of cells comprising cells selected from notochordal cells, notochordal derived cells, cells guided by notocordal cells, comprising contacting the population of said cells with viral inactivated-plasma cryoprecipitate concentrate (VIPCC) having an initial fibronectin/fibrinogen relative concentration of higher than 1/12 or about 1/11 to about 1/5.

In an embodiment of the invention, the VIPCC is activated VIPCC. In another further embodiment of the invention, said contacting is carried out ex vivo. Yet in another embodiment of the invention, said contacting is carried out in vivo.

In a further embodiment of the invention, the population comprises Nucleus Pulposus cells. The population can further comprise Annulus Fibrosus cells.

Yet in another aspect of the invention, the invention provides a method for treating an intervertebral disc and/or central nervous system disease, disorder or condition comprising administering to a subject in need a kit, a scaffold, a tissue or cell bank and/or a vehicle according to the invention.

The fibrin glue according to the invention can also be used as a method for treating an intervertebral disc and/or central nervous system disease, disorder or condition comprising injecting into the disc of a subject in need a viral inactivated-plasma cryoprecipitate concentrate (VIPCC), optionally in combination with a cell composition comprising cells selected from notochordal cells, notochordal derived cells, and cells guided by notocordal cells, wherein the cryoprecipitate comprises an initial fibronectin/fibrinogen relative concentration of higher than 1/12 or a about 1/11 to about 1/5.

In one embodiment of the invention, the VIPCC is activated VIPCC. In another embodiment of the invention, the VIPCC comprises an initial fibronectin/fibrinogen relative concentration of about 1/10 to about 1/5. In another further embodiment of the invention, the VIPCC comprises a contrast agent. The contrast agent can be iodine.

The method according to the invention can be used for preventing intervertebral disc herniation. In another embodiment of the invention, the subject in need is at early stage of intervertebral degenerative disease. Yet in another further embodiment of the invention, the activated VIPCC serves as a scaffold for reconstruction of Nucleus Pulposus in a subject at advanced stage of intervertebral degenerative disc disease.

The activated VIPCC obtainable according to the invention can serve as a scaffold for reconstruction of injured or ruptured spinal cord.

The method according to the invention can comprises injecting the VIPCC in combination with a cell composition comprising cells selected from notochordal cells, notochordal derived cells, and cells guided by notocordal cells.

In an embodiment of the invention, the method comprises excising all or a portion of the Nucleus Pulposus from the intervertebral disc prior to injecting the VIPCC and the cells.

In another embodiment of the invention, the notochordal cells and/or notochordal derived cells are from Nucleus Pulposus tissue excised from the intervertebral disc.

In another further embodiment of the invention, prior to the injection the cells were cultured ex-vivo on VIPCC comprising a fibronectin/fibrinogen ratio of about 1/11 to about 1/5.

The kit, scaffold, tissue or cell bank and/or the vehicle according to the invention can be used in the manufacture of a medicament for treating intervertebral degenerative disc and/or central nervous system injury such as spine injury.

The viral inactivated-plasma activated cryoprecipitate concentrate, which comprises an initial fibronectin/fibrinogen relative concentration of higher than 1/12 or about 1/11 to about 1/5 can be used for the manufacturing of a medicament for treating an intervertebral degenerative disc, and central nervous system disease, disorder or condition.

### Brief description of the drawings

The features, aspects, and advantages of the present invention will become better understood with regard to the following description, examples, claims, and the following figures.
**Fig 1****:** shows the effect of thrombin, viral inactivated-plasma cryoprecipitate concentrate (VIPCC) and components thereof on attachment and proliferation of Annulus Fibrosus cells. A 97-mm plastic culture dish was coated at the circumference with VIPCC, thrombin, albumin and fibrinogen. Afterwards, Annulus Fibrosus cell suspension was seeded in the center of the dish (Origin). Cell attachment to VIPCC, thrombin, albumin and fibrinogen was determined after 14 days by Hematoxylin and Eosin staining.
**Fig 2****:** shows the effects of trypsinization procedure and/or passage on cell attachment and proliferation of Annulus Fibrosus cells. Dissociated Annulus Fibrosus cells were placed in the center of a 97-mm plastic culture dish coated at the circumference with VIPCC, fibrinogen, fibronectin, and purified fibronectin/fibrinogen mixture (1:10). Cell attachment was assessed after 9 days by Hematoxylin and Eosin staining.
**Figs 3A-3B****:** show Nucleus Pulposus (A) and Annulus Fibrosus (B) cell-attachment on different coatings. The results obtained are expressed as a fold of fibronectin cell attachment in the same experiment (100%). Fn-fibronectin; Fgn-fibrinogen.
**Fig 4****:** shows quantification of Nucleus Pulposus cells on different coatings on day 1 and 5 in culture. The results are presented as percentage of fibronectin absorbance intensity (100%). VIPCC- viral inactivated-plasma cryoprecipitate concentrate; Fn-fibronectin; Fgn-fibrinogen.
**Fig 5****:** shows Nucleus Pulposus cell-attachment on constant amount of fibronectin and increased amount fibrinogen in the coating matrix (A) and on constant amount of fibrinogen and increased amount fibronectin in the coating matrix (B).
**Figs 6A-6D****:** show the morphology of the Annulus Fibrosus (upper panel) and Nucleus Pulposus chondrocytes (bottom panel) in attached (A,C) and detached (B,D) three-dimensional scaffold prepared with VIPCC.
**Fig 7****:** Nucleus Pulposus or Annulus Fibrosus cell suspensions were prepared using different dilutions of VIPCC (1:2.5, 1:5 and 1:10). The suspension was mixed with an equivalent volume of thrombin component. The final cell suspension (30, 60 or 90 µl) was placed in the center of a 24-well plate culture dish. After a clot was formed, 20 fold diluted VIPCC and thrombin mixture were added at the circumference. The Fig shows the localization of the Nucleus Pulposus chondrocytes in a 60 µl volume of 10 fold diluted VIPCC (left panel) and in a 30 µl volume of 20 fold diluted VIPCC (right).
**Fig. 8****:** shows a fluorography image of control (A; saline) and activated-VIPCC (B; prepared with VIPCC and thrombin) injections into the intervertebral disc.
**Fig. 9****:** shows the isolated lumber spine 14 days after the injection procedure described in Fig. 8.
**Fig. 10**: shows decalcified intervertebral discs (IVDs) of control (A) and activated-VIPCC (B) injections described in Fig. 8.
**Fig. 11**: shows histological studies of the Nucleus Pulposus region of control (A) and activated-VIPCC (B) IVDs injections described in Fig. 78.
**Fig. 12**: shows the central region of the Nucleus Pulposus of control (A) and activated-VIPCC (B) IVDs injections described in Fig. 8.
**Fig. 13****:** shows clusters of Nucleus Pulposus in the peripheral region of control (A) and activated-VIPCC (B) IVDs injections.

### Detailed description of the invention

The invention relates to the use of viral inactivated-plasma cryoprecipitate concentrate (VIPCC) comprising a suitable fibronectin/fibrinogen ratio for treating, regenerating, repopulating injured Nucleus Pulposus cells/tissue, notochord derived tissue, and/or tissue whose formation is guided by notochordal cells, e.g. spinal intervertebral disc and/or central nervous system such as spine.

The cells of human Nucleus Pulposus tissue are primarily small chondrocyte-like cells, but there is a second population of large cells, the notochordal cells, which are presumed remnants of the embryonic tissue that guided formation of the embryonic development of the neural tube and the Nucleus Pulposus (Hunter et al, 2003). Some studies indicate that notochord cells found in the Nucleus Pulposus tissue can guide the formation of Nucleus Pulposus and CNS cells and/or can be used as progenitors of such cells. Therefore, Nucleus Pulposus cells can be used to assist in reconstruction of the intervertebral disc and the central nervous system (CNS).

It was surprisingly found according to the present invention that fibronectin/fibrinogen ratio plays a crucial role in Nucleus Pulposus cell attachment, proliferation and migration. It has been shown according to the invention that (chondro) conductivity properties of cryoprecipitate on the Nucleus Pulposus (NP) absolutely depend on an optimal ratio of fibronectin relative to fibrinogen.

The term "cryoprecipitate" refers to a human blood component which is obtained from frozen plasma prepared from whole blood. A cryoprecipitate can be obtained when frozen plasma is thawed in the cold, typically at a temperature of 0-4°C, and collecting the precipitate , for example by centrifugation. Usually a cryoprecipitate is formed which is rich in fibrinogen, factor VIII, von Willebrand factor, factor XIII, and fibronectin.

For example, the results show that an increase in fibrinogen concentration significantly decreased Nucleus Pulposus cell attachment. While an increase in fibronectin concentration lead to the opposite outcome. On the other hand cryoprecipitate which is selectively depleted of fibrinogen and composing high levels of fibronectin would not cure, and therefore without fibrinogen a scaffold which supports a three-dimensional tissue formation will not form.

It was found that a purified mixture of fibronectin/fibrinogen in a ratio of 1/10 and 1/5 is more suitable for cell-attachment and proliferation of Nucleus Pulposus cells than mixtures comprising a lower fibronectin/fibrinogen ratio. Therefore cryoprecipitate containing optimal ratio of fibronectin/fibrinogen are superior over a cryoprecipitate not containing fibronection or comprising a low ratio of fibronectin/fibrinogen. Thus a viral-inactivated human plasma concentrated cryoprecipitate (VIPCC) having a fibronectin/fibrinogen ratio higher than 1/12, or from about 1/11 to about 1/5, or from about 1/10 to about 1/5 can be advantageously used to stimulate attachment, proliferation and migration of cells from Nucleus Pulposus tissue. Since Nucleus Pulposus originate from and/or are guided by notochordal cells, these results pave the way to new approaches for therapy of injured tissue derived from notochordal cells or tissue whose formation is guided by notochordal cells such as intervertebral disc and motor neurons from central nervous system.

Thus, the invention relates to a method for enhancing growth, proliferation, differentiation, maintenance, repair and/or restoration of a population of cells comprising cells selected from notochordal cells, notochordal derived cells, cells guided by notocordal cells, the method of which comprises contacting the population of said cells with viral inactivated-plasma cryoprecipitate concentrate (VIPCC) having an initial fibronectin/fibrinogen relative concentration of higher than 1/12 or about 1/11 to about 1/5.

In one aspect, the invention relates to a method for treating and/or preventing an intervertebral disc disease, disorder or condition comprising injecting into the disc of a subject in need a viral-inactivated human plasma concentrated cryoprecipitate (VIPCC) having a fibronectin/fibrinogen ratio higher than 1/12, from about 1/11 to about 1/5, or from about 1/10 to about 1/5.

In another aspect, the invention provides a method useful for treating central nervous system (CNS) disease or injury comprising administering to the site of the injury of a subject in need a viral-inactivated human plasma concentrated cryoprecipitate (VIPCC) having a fibronectin/fibrinogen ratio higher than 1/12, from about 1/11 to about 1/5, or from about 1/10 to about 1/5. In one embodiment, the invention is useful for treating injury of the CNS or the spinal cord, and enhances CNS restoration and axonal growth in a subject.

The term progenitor cell is used in cell biology and developmental biology to refer to immature or undifferentiated cells, typically found in post-natal animals. The majority of progenitor cells lie dormant or possess little activity in the tissue in which they reside. They exhibit slow growth and their main role is to replace cells lost by normal attrition.

As used herein the term "central nervous system disease or injury" refers to any disease, disorder, or trauma that disrupts the normal function or communication of the brain or spinal cord.

The injection procedure can be carried as follows. The subject in need can be positioned laterally and bended forward. The needle or cannula can be positioned directly adjacent to the Annulus Fibrosus of the disc to be treated.

When treating a central nervous system defect, the needle or cannula can be positioned adjacent to the dura.

The needle or cannula can be inserted under the guidance of a tracer. The technique which may be used in the method of the invention includes fluorography, scanning, magnetic resonance imaging, tomography, nanotechnology, digital video, X-ray or any other technique known in the art.

Non limiting examples of tracers are organic dyes, food dyes and/or fluorescent dyes. The contrast agent may be chosen from various non-toxic agents, such as iodine. If the spectrum of the tracer is not visible to the human eye the tracer can be detected by appropriate equipment.

Once in proper position, the needle or cannula can be inserted into the Nucleus Pulposus after traversing the Annulus Fibrosus.

Then the contrasting agent is injected in order to verify the exact application site. The contrast agent can be present at the end of the tip of the needle or cannula.

Non limiting examples of contrast agents are organic dyes, food dyes and/or fluorescent dyes. The contrast agent may be chosen from various non-toxic agents, such as iodine. If the spectrum of the contrast agent is not visible to the human eye the agent can be detected by appropriate equipment.

Once the location is verified the VIPCC and an equal volume of activating component, which is capable to form fibrin, are injected into the intradiscal space. The injection procedure can be in any order, for example, the components are applied simultaneously and a scaffold is formed when the components are mixed. The activating component can be thrombin and/or a solution obtainable from snake venom.

The activated VIPCC can be combined with a cell composition comprising notochordal derived cells such as Nucleus Pulposus cells.

The activated VIPCC required for the reconstruction of the damaged area will vary and is dependent on the extent of the injury.

The components can be injected at the base of the defect and the needle or cannula can be withdrawn from the Annulus Fibrosus when encountered resistance. Excess solutions can be spilt out of the injection site, forming a solid gel, forming a closure of the aperture or incision following the injection.

In one embodiment of the invention, the VIPCC comprises a fibronectin/fibrinogen ratio of about 1/7. The term "viral-inactivated human plasma concentrated cryoprecipitate" relates to a cryoprecipitate of whole blood having a fibronectin/fibrinogen ratio higher than 1/12, for example as described in EP patent 534,178, and obtainable by
- thawing a cryopaste;
- dissolving in a buffer at pH 7.0 to 7.2;
- preheating to 30 to 35°C;
- adjusting pH to 7.0 to 7.2;
- adding aluminum hydroxide under stirring;
- centrifuging and discarding the precipitate;
- adding CaCl₂;
- virus inactivation;
- concentrating by ultrafiltration to a protein concentration of 60 to 100 mg/ml.

Currently, fibrin glue or fibrin formulations have been disclosed for use in intervertebral disc and central nervous system regeneration, however, we found according to the present invention that cryoprecipitate, such as the one prepared according to the Martinowitz and Bal method, which contains a fibronectin/fibrinogen ratio close to the ratio of the original cryoprecipitate is more suitable for enhancing adhesion, proliferation, growth, differentiation, and/or maintenance of cells from Nucleus Pulposus tissue than compositions comprising a lower fibronectin/fibrinogen ratio.

Other commercial formulations such as TISSEEL^{®}, Tissucol® have a lower fibronectin/fibrinogen ratio than the original cryoprecipitate, thus the cryoprecipitate of the invention comprising a ratio of more than 1/12, from about 1/11 to about 1/5, from about 1/10 to about 1/7 and of about 1/7 , for example BAC; Omrix, IL prepared as described in EP Patent 534,178, wherein the plasmin(ogen) is removed as described in EP patent 1,390,485, is more suitable for intervertebral disc and central nervous system regeneration than other commercially available cryoprecipitate e.g. cryoprecipitate that are based on Schwartz et al methodology and which do not retain the fibronectin/fibrinogen ratio present in the original cryoprecipitate or have a ratio equal or lower than 1/12 (Bar et al, 2005).

The cryoprecipitate according to the present invention contains certain substances and specific fibronectin/fibrinogen ratio that stimulate attachment, migration and proliferation of notochordal cells or notochordal derived cells, a phenomenon which may facilitate in reconstitution of degenerative intervertebral disc and central nervous system.

The cryoprecipitate according to the invention can comprise a stabilizing agent, for example, tranexamic acid and arginine or lysine or mixtures of arginine and lysine, or their pharmaceutically acceptable salts. The solution of cryoprecipitate comprises a mixture of proteins such as fibrinogen, Factor VIII, Factor XIII, fibronectin, von Willebrand factor (vWF), vitronectin and the like. The solution of cryoprecipitate can comprise a protease inhibitor. In one embodiement, the protease inhibitor is aprotinin. Such cryoprecipitate is described in US Patent 6,121,232. In another embodiment of the invention, the cryoprecipitate can be free of plasminogen and plasmin as described in US Patent 7,125,569. The virus inactivation procedure that can be used is described in PCT/EP 91/00503. The basic principle is treatment of the cryoprecipitate with special detergents and removing the detergent later on from the cryoprecipitate. In one embodiment of the invention, the cryoprecipitate is double viral inactivated. According to the invention the cryoprecipitate is a concentrated cryoprecipitate. The cryoprecipitate is concentrated in the range of about a factor of 2 to about a factor of 5. In one embodiment of the invention, the concentration factor is of about 3. In an embodiment of the invention, the cryoprecipitate is concentrated by ultrafiltration to a protein concentration of 60 to 100 mg/ml. In a preferred embodiment of the invention, the VIPCC is for injection to the spine and does not contain tranexamic acid.

In one embodiment of the invention, the human plasma cryoprecipitate is activated. In another further embodiment of the invention the cryoprecipitate is non-activated. The activation procedure can be achieved by mixing said cryoprecipitate with an equivalent volume of an enzyme which is capable to react with fibrinogen to form fibrin. In one embodiment of the invention, the enzyme is thrombin and/or a solution obtainable from snake venom. The thrombin can be isolated from plasma of mammals such as bovine and is typically isolated from human beings. It is also possible that the enzyme which is capable to form fibrin is prepared by recombinant methods. The cryoprecipitate according to the invention and/or the enzyme capable to form fibrin can be supplied as a solution or in a solid form, for example as a lyophilized powder. The solution can be in frozen state.

The activated cryoprecipitate which has the bio-mechanical and physiological properties of the Nucleus Pulposus can be advantageously used to replace damaged natural tissue of the Nucleus Pulposus.

The cryoprecipitate can comprise a contrast agent. A "contrast agent" refers to a tracer which is able to make it possible to visualize the anatomy of the intervertebral disc. The technique which may be used in the method of the invention includes scanning, magnetic resonance imaging, tomography, nanotechnology, digital video, X-ray or any other technique known in the art.

Non limiting examples of contrast agents are organic dyes, food dyes and/or fluorescent dyes. The contrast agent may be chosen from various non-toxic agents, such as iodine. If the spectrum of the contrast agent is not visible to the human eye the agent can be detected by appropriate equipment.

The present invention discloses formation of a two- or three-dimensional scaffold for in vivo and/or in vitro applications including a biocompatible implant for tissue engineering in vivo, as well as for in vitro culturing for cells. The term "tissue engineering" typically refers to the use of a combination of suitable biochemical and physio-chemical factors to restore, replace, maintain, and/or enhance tissue function or a whole organ. The term tissue engineering sometimes is synonymous with the term regenerative medicine.

The term "scaffold" generally refers to a matrix which is able to provide structural integrity and supports a three-dimensional tissue formation, thereby allowing tissue reconstruction.

The scaffolds which may be used in the method of the invention should have at least one of the following properties: non-toxic, biocompatible, biodegradable, allow cell attachment and migration, deliver and retain cells and biochemical factors and, enable diffusion of vital cell.

In one embodiment of the invention, said activated cryoprecipitate which is capable of curing in vivo and form an artificial scaffold is injected into the degenerate disc or the injured CNS of a subject in need. The utilization of the in vivo cured cryoprecipitate is for providing mechanical support and anchoring source to a defective or injured site in situ and/or for providing a matrix into which cells from the injured site can migrate, invade, grow, proliferate, and/or differentiate.

As used herein the term "curing" refers to a composition which is curable in the presence of a curing catalyst to form a scaffold which has the bio-mechanical and physiological properties to support a three-dimensional tissue formation. "A curing catalyst" as defined herein refers to any agent that can react with the matrix pre-polymer resulting in solidification, for example, cross-linking or gelling, of the composition. Curing agents include cross-linking agents and agents that cure the composition in any other way. The curing agents can be chosen from the group of curing agents that are suitable to react with the matrix pre-polymer. In one embodiment of the present invention, the catalyst is an enzyme, e.g. transglutaminase. In another embodiment of the invention, the catalyst is Factor XIII.

The CNS injuries which can be treated according to the present invention are diverse and will be easily understood by the skilled person. Without limitation, there may be mentioned brain and spinal cord injuries due to neurosurgery, trauma, ischemia, hypoxia, neurodegenerative disease, metabolic disorder, infectious disease, compression of the inervertebral disc, tumors and autoimmune disease. In one embodiment of the invention, the in vivo cured cryoprecipitate is administering along the injured spinal cord for the reconstruction of injured or rapture spinal cord caused, for example, by compression of the inervertebral disc.

The in vivo cured cryoprecipitate is useful for reconstitution of degenerated intervertebral disc at different developmental stages. In one embodiment of the invention, the method according to the invention is used for preventing intervertebral disc herniation. In another embodiment of the invention, the method according to invention is for a subject at an early stage of intervertebral disc disease to seal small tears in the Annulus Fibrosus before herniation takes place. In a further embodiment of the invention, the cured cryoprecipitate serves as a scaffold for reconstruction of Nucleus Pulposus in a subject at advanced stage of intervertebral disc disease and/or spinal cord injury caused by compression or herniation of intervertebral disc.

Alternatively, the method according to the invention comprises the step of excising all or a portion of the Nucleus Pulposus prior to injecting said cryoprecipitate. The removal procedure can be carried out enzymatically by disrupting the extracellular matrix, mechanically, by using a Nucleotome probe and/or by any other method known in the art.

For example, surgery can be carried out while the patient is deep asleep or pain free by for example general or local anesthesia respectively. An incision can be carried out over the site of degeneration, typically the lower back, in the midline. The bone that curves around and covers the spinal cord (lamina) can be removed (laminectomy) and the tissue that is causing pressure on the nerve or spinal cord can be removed. The hole through which the nerve passes can be enlarged to prevent further pressure on the nerve. Sometimes, a piece of bone (bone graft), interbody cages, or pedicle screws may be used to strengthen the area of surgery. The cryoprecipitate of the invention can be introduced trough the hole and/or can be used to coat means used for strengthen the area of surgery.

In one embodiment of the invention, the patient receives local anesthesia and the procedure is a minimal invasive procedure.

Non limiting examples of mechanical separation techniques include mincing, chopping, slicing, milling, pulverizing, shearing, grinding, trimming, stripping, skinning. Disc cell isolation can be further facilitated with the use of other known separation techniques, such as filters, centrifuges, separation columns, or affinity columns.

The proteolytic enzymes capable of degrading cartilage tissue include, but are not limited to; serine peptidases, for example, trypsin, chymotrypsin, pancreatic elastase; cystein peptidases, for example, papain chymopapain; aspartic peptidases, for example, pepsin, metallo peptidases, for example, collagenase, gelatinase, pronase, chondroitinase; hyaluronidase and/or alternative chemical materials which would degrade disc material in the same or similar manner, and combinations thereof.

It should be understood that the amount of the pharmacologically suitable solution of enzymes required for the degradation of mammalian disc tissue will vary.

The term "pharmacologically suitable solution" refers to dissolving an effective amount of proteolytic enzymes in a solution. The pH of the solution can be adjusted to a physiologically compatible pH of about 7.40 for maximum activation of the proteolytic enzymes. For better results, the solution will contain all components required to activate the proteolytic enzymes.

The proteolytic enzymes capable of degrading cartilage tissue can be supplied as a lyophilized powder and/or in a solid form. The solution and/or the powder are sterile, pyrogen-free in a freeze dried state until immediately prior to use. The vial, which contains the lyophilized powder, is allowed to warm to room temperature and immediately reconstituted in a sterile aqueous solution.

As used herein, the term "pyrogen" refers to infective particle, such as a virus, a prion, an endotoxin and/or an exotoxin, which can infect or propagate in cells of a biological organism.

The patient can be positioned laterally and bended forward and the needle or cannula can be positioned selectively in the Nucleus Pulposus under guidance, for example, scanning, magnetic resonance imaging, tomography, nanotechnology, digital video and/or X-ray, or by any other technique known in the art.

Non limiting examples of guiding agents are non-toxic organic dyes, food dyes and/or fluorescent dyes. If the spectrum of the guiding agent is not visible to the human eye the agent can be detected by appropriate equipment.

Once the location is verified, the proteolytic enzymes solution can be injected into the injured area in order to degrade all or a portion of the Nucleus Pulposus tissue. The digested tissue can be aspirated and placed in a tube. Growth medium such as DMEM/ Ham's F12 medium can be added into the suspension.

In another embodiment of the invention, Nucleus Pulposus tissue is excised using a Nucleotome probe. The patient can be positioned laterally and bended forward and the cannula containing the Nucleotome probe can be inserted under guidance as specified above. The Nucleotome probe has a rounded tip which can resect and aspirate Nucleus Pulposus tissue from the lumbar disc. The collected Nucleus Pulposus tissue can be partially or completely digested at 37°C with proteolytic enzyme solution capable of degrading cartilage tissue as specified above.

The activated VIPCC of the invention that can cure in vivo can be injected with the harvested Nucleus Pulposus cells, thereby providing an effective scaffold in which the injected cells can grow in three-dimensional mode.

In the case that Nucleus Pulposus cells are treated with proteolytic enzyme, prior to the injection procedure, the proteolytic enzyme solution can be flushed from the dissociated Nucleus Pulposus cells and/or tissue by adding an effective amount of inactivators and/or removing the proteolytic enzymes by centrifugation and discarding the supernatant phase, or by any other method known in the art.

The harvested dissociated Nucleus Pulposus cells can be frozen in liquid nitrogen and then stored at -80 °C until use.

Alternatively, the method according to the invention may include, without excluding other possibilities, autologous, allogenic, xenogenic and/or cells harboring recombinant DNA.

The term "autologous" cells refer to cells originally derived from the same individual to which they will be re-implanted.

The term "allogenic" cells refer to cells obtained from the body of a donor of the same species.

"xenogenic" cells are those isolated from individuals of another species.

The injected cells can be a population of cells comprising cells selected from notochordal cells, notochordal derived cells, cells guided by notocordal cells.

Thus, the population of cells, such as Nucleus Pulposus cells, and the cryoprecipitate of the invention can be injected to the NP and advantageously the latter will also seal fissures in the Annulus Fibrosus.

The population of cells may further comprise Annulus Fibrosus cells (e.g. from cadaver).

In one embodiment of the invention, the injected cells are from Nucleus Pulposus. In a further embodiment of the invention, the cells are from autologous origin. In another further embodiment of the invention, the removal of autologous Nucleus Pulposus, and cell injection or re implantation steps are performed within the same surgical procedure.

The cells to be injected in the subject in need can be included in the activating component e.g. thrombin, in the cryoprecipitate component, and/or can be in a separated component.

According to one embodiment of the present invention, the injected cells can be cultured ex-vivo prior to the injection procedure on a cryoprecipitate comprising a fibronectin/fibrinogen ratio higher than 1/12. In another embodiment of the invention, the ratio is from about 1/11 to about 1/5. In another further embodiment of the invention, the cryoprecipitate contains a fibronectin/fibrinogen relative concentration of from about 1/10 to about 1/7. Yet in another further embodiment of the invention the fibronectin/fibrinogen ratio is of about 1/7.

The ex-vivo cultured cells can be grown on activated and/or non-activated cryoprecipitate. The activation procedure can be achieved by mixing said cryoprecipitate with an enzyme which is capable to react with fibrinogen to form fibrin. In one embodiment of the invention, the enzyme is thrombin and is added in an equivalent volume to the cryoprecipitate. In another embodiment of the invention, the enzyme is a solution obtainable from snake venom. The thrombin can be isolated from plasma of human beings or mammals such as bovine and/or prepared by recombinant methods. The cryoprecipitate of the invention and/or the enzyme capable to form fibrin can be supplied as a solution or in a solid form, for example as a lyophilized powder. The solution can be in frozen state.

As used herein, "ex-vivo" cell culture refers to culturing cells outside of the body. Ex-vivo cell culture includes cell culture in vitro, e.g., in suspension, or in single or multi-well plates. Ex-vivo culture also includes co-culturing cells with different cell types, and culturing in or on two- or three-dimensional matrices.

In another aspect, the invention relates to a kit for injecting therapeutic solutions into a damaged spinal intervertebral disc comprising: a first container comprising cryoprecipitate according to the invention, and a second container comprising an enzyme capable of forming fibrin when it reacts with fibrinogen. In one embodiment of the invention, the fibronectin/fibrinogen ratio is higher than 1/12. In another embodiment of the invention, the ratio is from about 1/11 to about 1/5. In another further embodiment of the invention, the cryoprecipitate contains a fibronectin/fibrinogen relative concentration of from about 1/10 to about 1/7. Yet in another further embodiment of the invention the fibronectin/fibrinogen ratio is of about 1/7.

In another aspect, the invention relates to a kit which is directed for injecting therapeutic solutions in the emergency treatment of spine and cord injury or other mitigation of injured axons into a injured central nervous system comprising: a first container comprising cryoprecipitate according to the invention, and a second container comprising an enzyme capable of forming fibrin when it reacts with fibrinogen. In one embodiment of the invention, the fibronectin/fibrinogen ratio is higher than 1:12. In another embodiment of the invention, the ratio is from about 1:11 to about 1:5. In another further embodiment of the invention, the cryoprecipitate contains a fibronectin/fibrinogen relative concentration of from about 1:10 to about 1:7. Yet in another further embodiment of the invention the fibronectin/fibrinogen ratio is of about 1:7 to 1:5.

The spinal intervertebral and the central nervous system reconstruction kits can also comprise a contrast agent, for example, in order to localize the site of injection. The contrast agent can be included in the container comprising the cryoprecipitate component, in the container comprising the enzyme component and/or in a separated container. In one embodiment of the invention, the contrast agent is formulated with the cryoprecipitate. In another embodiment of the invention, the contrast agent is formulated with the enzyme capable of forming fibrin. Examples of contrast agents include, but are not limited to, organic dyes, food dyes and/or fluorescent dyes. The contrast agent may be chosen from various non-toxic agents, such as iodine. The techniques which may be used to detect the contrast agent include scanning, magnetic resonance imaging, tomography, nanotechnology, digital video, X-ray or any other technique known in the art. If the spectrum of the visualization agent is not visible to the human eye the agent can be detected by appropriate equipment.

The spinal intervertebral and the central nervous system reconstruction kits according to the invention can further comprise another container comprising proteolytic enzyme to degrade extracellular matrix such as proteolytic enzymes capable of degrading cartilage tissue that include, but are not limited to serine peptidases, for example, trypsin, chymotrypsin, pancreatic elastase; cystein peptidases, for example, papain chymopapain; aspartic peptidases, for example, pepsin; metallo peptidases, for example, collagenase, gelatinase, pronase chondroitinase; hyaluronidase and/or alternative chemical materials which would degrade disc material in the same or similar manner, and combinations thereof.

The cryoprecipitate and/or the enzyme capable of forming fibrin and/or the proteolytic enzyme and/or the contrast agent can be provided in the spinal intervertebral and the central nervous system reconstruction kits as a solution and/or in a solid form, for example, as lyophilized powder. The solution can be in frozen state. The kits can comprise instruction for use. The kits can also comprise a needle such as a spinal needle including for example a curved spinal needle. A spinal cannula may alternatively be used. A single, dual or multi-barrel syringe, or other fibrin sealant delivery device, may be included in the kit.

Subject matter of the present invention is also a scaffold suitable for repair and/or regeneration of spinal intervertebral disc and central nervous system when administered in the body. The scaffold is prepared using a cryoprecipitate according to the invention and an enzyme which is capable to react with fibrinogen to form fibrin. In one embodiment of the invention, the cryoprecipitate comprises a fibronectin/fibrinogen ratio higher than 1/12. In another embodiment of the invention, the ratio is from about 1/11 to about 1/5. In another further embodiment of the invention, the cryoprecipitate contains a fibronectin/fibrinogen relative concentration of from about 1/7 to about 1/10. Yet another further embodiment of the invention, the cryoprecipitate contains a fibronectin/fibrinogen ratio of about 1/7- 1/5.

The enzyme which is capable to form fibrin can be thrombin and/or a solution obtainable from snake venom. In an embodiment of the invention, the thrombin is isolated from plasma of human beings or mammals such as bovine. It is also possible that the enzyme which is capable to form fibrin is prepared by recombinant methods.

The components of the spinal intervertebral and the central nervous system reconstruction kits or formulation of the scaffolds can be in separated recipients such as syringes which can be applied in any order, for example, the components can be applied simultaneously or one after the other and a scaffold is formed when the components are mixed.

In one embodiment of the invention, the separated recipients can be configured for injection of said cryoprecipitate, an enzyme capable of forming fibrin, and notochordal or notochordal derived cells. In another embodiment of the invention, the notochordal or notochordal derived cells can be combined with said cryoprecipitate and/or with the enzyme capable of forming fibrin.

According to the invention, the components of the formulation can be provided as a solution and/or in a solid form. The solution can be in frozen state.

Another object of the invention is establishing a tissue or cell bank of notochordal or notochordal derived cells for use in repair and/or regeneration of intravertebral disc and central nervous system reconstruction comprising notochordal cells or notochordal derived cells grown in a scaffold as specified above. According to the invention the tissue or cell bank comprise autologous, allogenic, xenogenic and/or cells harboring recombinant DNA.

As used herein, "tissue or cell bank" refers to a tissue or cell bank developed by a method which allows the establishment of notochordal cells or notochordal derived cells suitable for repairing, regenerating and/or replacing damaged tissue, for example, intravertebral disc tissue and central nervous system tissue such as axons and motorneurons. The cells in said tissue or cell bank can maintain their characteristics, e.g, biochemical, morphological and/or physical properties, necessary to perform specialized function. The cells have the ability to proliferate and/or differentiate in or on two- or three - dimensional matrices, by culturing said cells alone or with other cell types.

Another aspect of the invention relates to a scaffold which can advantageously be used as a vehicle for delivering notochordal cells or notochordal derived cells into a damaged tissue such as intervertebral disc and central nervous system tissue because the cryoprecipitate according to the invention provides a suitable scaffold on which the notochordal or notochordal derived cells can attach, migrate, grow and divide. The vehicle comprises cryoprecipitate according to the invention and notochordal, notochordal derived cells and/or cell guided by notochordal cells. In one embodiment of the invention, the cryoprecipitate comprises a fibronectin/fibrinogen ratio higher than 1/12. In another embodiment of the invention the ratio is about 1/11 to about 1/5. In another further embodiment of the invention, the cryoprecipitate comprises a fibronectin/fibrinogen ratio of about 1/10 to about 1/7. Yet in another embodiment of the invention, the cryoprecipitate comprises a fibronectin/fibrinogen ratio of about 1/7.

The term "vehicle" or cell delivery vehicle refers to a means for delivery of cells into a specific tissue in vivo. Viable cells are incorporated into the scaffold according to the invention and administered into the damaged tissue such as disc or central nervous system allowing reconstitution repair or restoration of the damaged site. The delivered cells can be autologous, allogenic, xenogenic and/or cells harboring recombinant DNA.

Disc cells cultured in monolayer tend to de-differentiate and loose their typical cell phenotype (Benya and Shaffer 1982). According to the invention it has been found that Annulus Fibrosus cells and notochordal derived cells, e.g. Nucleus Pulposus cultured in cryoprecipitate of the invention maintain their phenotype and provide a matrix which can be used in an in vitro and in vivo expansion for reconstructive surgery of cartilage and central nervous system. It was found according to the present invention that VIPCC has chondroconductive components which stimulate cell attachment and proliferation of Annulus Fibrosus and Annulus Pulposus cells seeded in culture dish coated with VIPCC. The findings according to the invention show that no detectable morphological changes or damage was done during sub-culturing or trypsinization. Moreover, even though the cells migrated throughout the culture dish a differential attachment to VIPCC and fibronectin was observed. The results prove that fibronectin, recognized as an agent which promotes cellular adhesion, is a fundamental component in the VIPCC and needs to be at a specific ratio with fibronectin to stimulate the attachment and proliferation of chondrocytes.

Thus, it is also subject matter of the invention to provide a method for enhancing growth, proliferation, differentiation and/or maintenance of notochordal or notochordal derived cells comprising contacting a population of cells from the Nucleus Pulposus with cryoprecipitate according to the invention. In one embodiment of the invention, the cryoprecipitate comprises a fibronectin/fibrinogen ratio higher than 1/12. In another embodiment of the invention, the ratio is from about 1/11 to about 1/5. In another further embodiment of the invention, the cryoprecipitate contains a fibronectin/fibrinogen relative concentration of from about 1/10 to about 1/7. Yet in another further embodiment of the invention, the cryoprecipitate contains a fibronectin/fibrinogen ratio of about 1/7. The contacting of the Nucleus Pulposus with the cryoprecipitate can be carried ex vivo and/or in vivo. According to the present invention, the contacting procedure can comprise other cell types.

The human plasma cryoprecipitate can be activated or non-activated. The contacted cells can be autologous, allogenic, xenogenic and/or cells harboring recombinant DNA.

The cryoprecipitate of the present invention has conductive and inductive capabilities.

The conductive or inductive capability of said cryoprecipitate of the present invention can be determined following use of the cryoprecipitate and consequently assess enhancement of adhesion, migration, proliferation, and differentiation of the cultured Annulus fibrosus and notochordal derived cells. Assessments of these properties are carried out by any technique known in the art, for example, proliferation by using a haemacytometer and

Hematoxylin and Eosin staining, differentiation, by measuring chondroitin sulfate expression, migration by using migration test as exemplified below.

As used herein, the term "conductive" refers to the ability of the activated cryoprecipitate to serve as a scaffold on which specific cells can attach, grow, migrate, proliferate and/or differentiate, including "chondroconductive" or "neuroconductive" etc.

As used herein, the term "inductive" refers to the capacity of the cryoprecipitate to stimulate specific cells to attach, grow, migrate, proliferate and/or differentiate, including "chondroinductive", or "neuroinductive" etc.

Another term use for "conductive" is "chemotactic". The term "chemotactic" refers to physiological responses that result in a characteristic movement or orientation of cell towards a chemical stimulus.

An intradiscal or intrathecal injection may result in local and chronic inflammation and consequently in changes in the typical structure of the Nucleus Pulposus population, for example, necrotic damage, pyknotic nuclei and karyolysis. We have found according to the invention that this problem can be solved or diminished by including the cryoprecipitate according to the invention in the formulation to be injected to the degenerated disc and injured central nervous system.

The kit, the formulation, the scaffold, the tissue or cell bank and/or the vehicle according to the invention, can comprise one or more additives such as, but not limited to: therapeutic agents (such as antibiotics, anti-inflammatories), analgesics, antitumor drugs, growth factors, proteins, hormones, cartilage inducing factors, proteoglycans such as aggrecan, type I and II collagen, oxygen-containing components, enzymes and the like.

One or more of these components can be included in the cryoprecipitate component, in the activating component e.g. thrombin, and/or can be in a separated component.

The kit, the formulation, the scaffold, the tissue or cell bank and/or the vehicle to be administering to a subject in need for the reconstitution of the central nervous system can comprise Rho Kinase Inhibitors such as, for example, Cethrin®, Y-27632, C3 etc.

Yet another object of the invention is accomplished by providing a method for treating intervertebral disc and central nervous system disease, disorder or condition comprising administering to a subject in need a kit, a formulation, a tissue or cell bank and/or a vehicle according to the invention.

The kit, formulation, tissue or cell bank and/or vehicle according to the invention for preventing and/or treating intervertebral degenerative disc disease can optionally include autologous, allogenic, xenogenic Annulus Fibrosus cells and/or Annulus Fibrosus cells harboring recombinant DNA.

The disclosure of ranges in the description of the invention is easily understood by the skilled person. It means the disclosure of continuous values and figures between the limits of the ranges, including the limiting figures and values. For example, if a range is given of from 7 to 10, it is meant at least 7, 8, 9 and/or 10 with all combinations of intermediate sub ranges such as 7 and 8, 7-9, 7-10, or 8 and 9 or 8-10 and so on. The ranges given in the present invention are for example, 1/11 to 1/5, 1/10 to 1/5, and 1/7 to 1/5. Thus, the ranges include 1/11, 1/10, 1/9, 1/8, 1/7 and 1/6 and all combinations of intermediate sub ranges such as 1/6 and 1/5, 1/11-1/5, or 1/7 and 1/6 or 1/11 to1/7 and so on.

The disclosure of applications, patents and publications, cited above or below, is hereby incorporated by reference.

The following examples are illustrative but not limiting.

### Examples

### Example 1:

### Preparation of Annulus Fibrosus and Nucleus Pulposus chondrocytes.

This example illustrates preparation of chondrocytes cell culture. Pig lumbar spine was removed from disc level L1-L6 12 hours after slaughter (transported at 4°C) and sterilized with septal scrab for 10 min and soaked in 70% ethanol for 5 min. The muscle and other connective tissues were removed and each vertebral body was dissected in the middle so that isolated intervertebral discs (IVD) could be obtained. The isolated IVDs were then dipped in graduated cylinder containing PBS with 200 µg/ml sodium azide for a few seconds. Followed by dipping in sterile PBS containing 10 U/ml penecillin, 0.1 mg/ml streptomycin, 2.5µg/ml amphothericin, 50µg/µl gentamycin, 10 µg/ml vancomycin, 65.6 µg/ml cephalosporin and 10% fetal bovine serum (FBS). Specimens were then transferred to a sterile laminar flow and the remaining procedure was done under sterile conditions.

The isolated IVDs were cut in halves using a scalpel and the Nucleus Pulposus tissue was carefully separated and placed in a 97 mm cell culture dish. The Nucleus Pulposus tissue was rinsed three times in PBS supplemented with the above mentioned antibiotics and FBS to remove residual debris. Then, the Nucleus Pulposus tissue was subjected to gentle digestion by a collagenase solution (3 mg/ml Sigma cat No C-6885 in DMEM/Ham's F12 medium (1:1) (Biological industries) supplemented with 10 U/ml penicillin, 0.1 mg/ml streptomycin, 0.25 µg/ml amphothericin, 100 mg/µl gentamycin, 10 µg/ml vancomycin and 6.56 µg/ml cephalosporin) for 3h at 37°C in a humidified atmosphere of 5% CO₂.

Prior to the digestion procedure the collagenase solution was transferred through 0.45 µm filter (Millipore SLHV033RS).

The digest was filtered through sterile gauze and centrifuged at 800g for 5 min. After centrifugation, the supernatant phase was discarded, and cells were re-suspended in 2 ml of DMEM/Ham's F12 supplemented with 10 U/ml penicillin, 0.1 mg/ml streptomycin, 0.25 µg/ml amphothericin, 100 mg/µl gentamycin, 10 µg/ml vancomycin and 6.56 µg/ml cephalosporin.

Annulus Fibrosus tissue was removed from the isolated IVDs, placed in a 97 mm cell culture dish and rinsed two times in PBS containing 10 U/ml penicillin, 0.1 mg/ml streptomycin, 2.5µg/ml amphothericin, 50µg/µl gentamycin, 10 µg/ml vancomycin, 65.6 µg/ml cephalosporin and 10% FBS. Annulus Fibrosus tissue was minced to small pieces (3-5 mm) using a scalpel. The minced tissue was incubated overnight in 5% CO₂ at 37°C. After incubation, the tissue was digested with the collagenase solution (prepared as above-mentioned) in 5% CO₂ at 37°C for 5 hours. After digestion was completed, the cell suspension was transferred through sterile gauze. Afterwards, the suspension was centrifuged at 800g for 5 min and the pellet containing the Annulus Fibrosus chondrocytes was re-suspended in 2 ml of fresh DMEM/Ham's F12 medium (supplemented as mentioned above).

### Example 2:

### Effect of thrombin viral inactivated-plasma cryoprecipitate concentrate (VIPCC) and components thereof on Annulus Fibrosus cell attachment and proliferation.

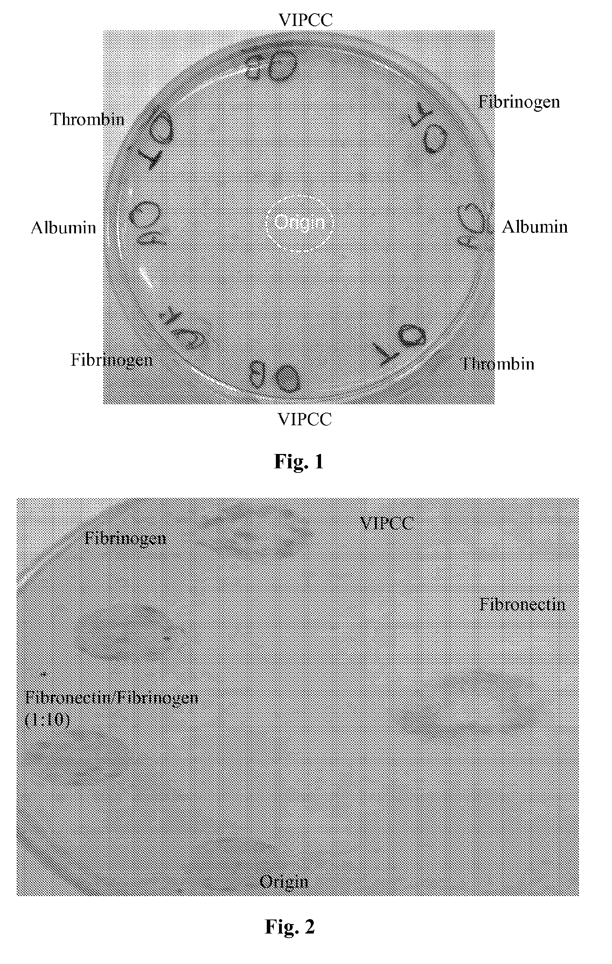

To investigate the effect of thrombin, VIPCC and components thereof on Annulus Fibrosus cell attachment and proliferation (chemotaxis), a 97-mm plastic culture dish (Fig. 1) was coated at the circumference with 80 µl of the following solutions containing 5-20 µg of total protein diluted in saline: VIPCC (BAC; Omrix, IL; prepared as described in EP Patent 534,178, wherein the plasmin(ogen) was removed as described in EP patent 1,390,485); human fibrinogen (Enzyme research; cat No FIB1 2800L); thrombin (Omrix, IL; prepared as described in US patent 5,143,838 and in EP patent 378,798); and human serum albumin (Sigma; cat No A7030). The fibrinogen content in the purified coating and in the VIPCC coating was about 8 µg. The solutions were let to dry in a laminar flow under sterile conditions. Afterwards, 150 µl Annulus Fibrosus cells (5X10⁶) suspended in growth medium (DMEM/ Ham's F12 supplemented with the above mentioned antibiotics and 10% FBS) were placed in the center of the dish (Fig. 1 Origin) and incubated overnight at 37°C in a 5% CO₂ atmosphere. After incubation, the medium was discarded and replaced by 10 ml growth medium (DMEM/Ham's F12 medium supplemented with antibiotics and 10% FBS), and the culture dish was incubated for additional 14 days.

Histological assessments of the above two dimensional cultures were performed by Hematoxilin and Eosin staining method. Briefly, the cultured chondrocytes were fixed with 95% ethanol for 15 min, washed in H₂O and exposed to Hematoxylin solution Gill NR1 (Sigma; cat No GHS116) for 3 min. Then the cultures were washed in H₂O, counter-stained with Eosin Y (Sigma; cat No E4382) for 30 seconds and rinsed in 95% Ethanol and H₂O. Afterwards, the cells were washed in H₂O three times and analyzed macroscopically and microscopically. As seen in Fig. 1, Annulus Fibrosus cells seeded at the center of a culture dish migrated evenly throughout the whole field, but showed better attachment and proliferation to matrix coated spots comprised of VIPCC as compared to uncoated or coated plates with human fibrinogen; thrombin; and human serum albumin. The results indicate that VIPCC has chondro-conductive components which stimulates Annulus Fibrosus cell attachment and proliferation and may facilitate in reconstruction of degenerative intervertebral disc.

### Example 3:

### Effect of trypsinization procedure and/or passage on Annulus fibrosus cell attachment and proliferation characteristics induced by VIPCC.

Passage number refers to the "age" of cell line or to the number of times the cells have been sub-cultured. Some cell lines may exhibit morphological changes after passages. In addition, cell culture procedures used for sub-culturing such as trypsinization can damage cell membranes resulting in poor attachment, clumping or "ragged" looking membranes. The present study was aimed to determine the effects of trypsinization and/or passage on cultured Annulus Fibrosus chondrocytes cell attachment mediated by VIPCC. For this purpose, chondrocytes derived from the Annulus Fibrosus were isolated and cultured in an uncoated 24-well plate as described in Example 1. The culture was incubated for 11 days in a humidified atmosphere of 5% CO₂ at 37°C. After incubation, the cells were enzymatically dissociated by adding 100 µl trypsin (Trypsin-EDTA Biological Industries, 03-050-1A) to each well at 37°C for 5 min. The dissociated chondrocytes were collected from three wells into a vial and the cell suspension was centrifuged at 800 *g* for 5 min. The supernatant phase was discarded, and the cells were re-suspended in 200 µl DMEM/Ham's F12 medium (supplemented with antibiotics and 10 % FBS) and placed in the center of a 97-mm plastic culture dish coated at the circumference with 80 µl drops of the following solutions: VIPCC containing 6.4 µg total protein (from which 4.7 µg is fibrinogen), 3 µg purified human fibrinogen, 0.18 µg fibronectin (produced according to Miekka et al. 1982) , and a purified fibronectin/human fibrinogen mixture in a concentration of 0.3:2.7 µg, respectively (approx. ratio of 1/10). The culture was incubated in 5% CO₂ at 37°C for 24 hours, washed twice in growth medium to remove unattached cells, 10 ml of DMEM/Ham's F12 medium supplemented with antibiotics and 10 % FBS was added and incubated for additional 9 days. Morphological assessment was performed by staining cells with Hematoxylin and Eosin solutions, followed by macroscopic and microscopic observation.

The results show that no morphological changes or damage was done to the Annulus Fibrosus cells during sub-culturing or trypsinization. Moreover, even though the cells migrated throughout the culture dish a differential attachment to VIPCC and fibronectin was observed (Fig.2). The results prove that fibronectin, recognized as an agent which promote cellular adhesion, is a fundamental component in the VIPCC which stimulates the attachment and proliferation of chondrocytes to the VIPCC.

### Example 4:

### Effect of fibrinogen/fibronectin ratio on Annulus Fibrosus and Nucleus Pulposus cell-attachment and proliferation.

The above example indicates that fibrinogen/fibronectin ratio affects chondrocyte migration proliferation and attachment. The present experiment was designed to investigate the effect of different ratios of fibronectin/fibrinogen mixtures on attachment and proliferation of cells from Nucleus Pulposus and Annulus Fibrosus. A twenty-four-well plate was coated with 200 µl of one of the following components: 0.626 µg purified fibronectin; 10 µg purified human fibrinogen (fgn); or with fibronectin and fibrinogen mixture in concentration of 0.626 µg: 3.13 µg, 0.626 µg: 6.26 µg, and 0.626 µg: 12.52 µg (ratios of 1/5, 1/10 and 1/20, respectively).

The solutions were let to adhere to the wells in a laminar flow under sterile conditions. After 3 hours, excess solution was removed and the plate was turned upside down to dry for an additional 2 hours. Freshly isolated Nucleus Pulposus or Annulus Fibrosus cells were suspended in DMEM/Ham's F12 medium (supplemented with the above mentioned antibiotics and 10 % FBS) and seeded into the above coated wells at a concentration of 6 X 10⁴ cell per well. The cultures were incubated in a humidified atmosphere of 5% CO₂ at 37°C.

Histological assessments of the above two-dimensional cultures were performed after 5 days in vitro. The cultured chondrocytes were exposed to Hematoxylin and Eosin Y solutions as specified above. Afterwards, the culture was stained with Crystal Violet (1 g in 100 ml of Acetic Acid) for 10 min and washed in H₂O.

In order to quantify the level of cell attachment, the color was extracted from the cells with 100 µl 70% ethanol for 10 min. 75 µl aliquots were transferred to a 96-well plates and the absorbance was measured in a spectrophotometer at a wavelength of 590 nm. Color extraction from cell attachment in the fibronectin coated cultures was considered 100%.

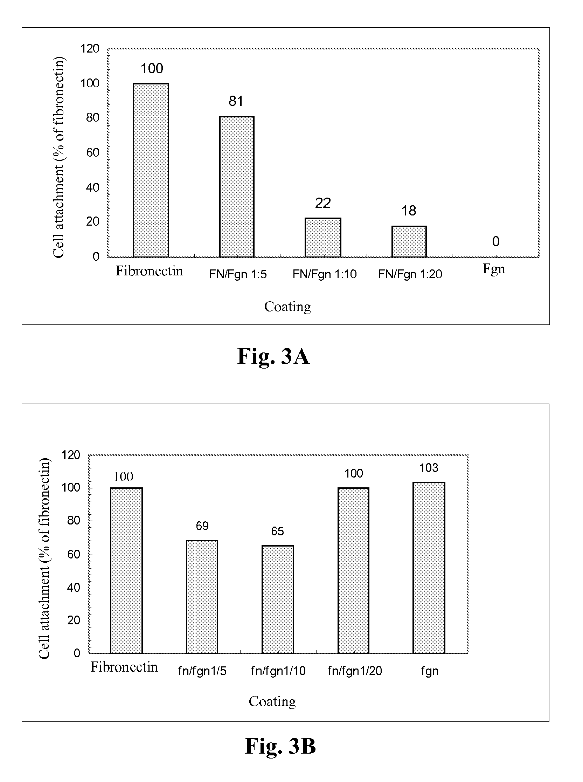

Results of Nucleus Pulposus and Annulus Fibrosus cell-attachment on the different coatings are presented in Fig 3A and B, respectively. The results show that an increase in fibrinogen concentration significantly decreased Nucleus Pulposus cell attachment. While an increase in fibronectin concentration lead to the opposite outcome (Fig 3A). These results indicate the key role of fibronectin in Nucleus Pulposus cell attachment.

Surprisingly, it was found that the Nucleus Pulposus cell attachment was more affected by the different coatings compared to Annulus Fibrosus chondrocytes (Fig 3B).It was found that fibronectin/fibrinogen ratio plays a crucial role in Nucleus Pulposus cell attachment. As seen in Fig 3A, a purified mixture of fibronectin/fibrinogen in a ratio of 1/5 showed a higher rate of Nucleus Pulposus cell attachment as compared to purified mixtures in ratios of 1/10 and 1/20. These results demonstrate that fibronectin/fibrinogen in a ratio of about 1/5 is more suitable for cell-attachment and proliferation of Nucleus Pulposus cells than mixtures comprising a lower fibronectin/fibrinogen ratio.

The efficacy of VIPCC (which contains a fibronectin/fibrinogen ratio in the range of 1/10-1/5) and fibronectin/fibrinogen mixture (1/10) in promoting cell proliferation level in Nucleus Pulposus and Annulus Fibrosus chondrocytes was evaluated in cells attached on day 12 compared to cells attached on day 5. The results are summarized in Table 1:

**Table 1: Nucleus Pulposus and Annulus Fibrosus cell proliferation level on VIPCC and fibronectin/fibrinogen mixture (1/10).**

| | | **fn/fgn (1/10) cell proliferation (%)** | **VIPCC cell proliferation (%)** |
|---|---|---|---|
| | Day of culture | | |
| **Nucleus Pulposus** | 5 | 22 | 56 |
| | 12 | 14 | 77 |
| **Annulus Fibrosus** | 5 | 65 | 62 |
| | 12 | 71 | 88 |

The results are presented as percentages relative to fibronectin cell attachment in the same experiment which was considered 100%.

The results indicate that Annulus Fibrosus cell culture seeded on VIPCC coating exhibited a significant increase in cell number (62 and 88% on day 5 and 12, respectively; Δ=26%) as compared to fibronectin/fibrinogen mixture coating (65 and 71% on day 5 and 12, respectively; Δ=6%). Nucleus Pulposus cell culture showed similar results, VIPCC coating resulted in an increase of 21% in cell number (56 and 77 % on day 5 and 12, respectively; Δ=21%), whereas fibronectin/fibrinogen mixture coating resulted in a decrease of cell attachment (22 and 14% on day 5 and 12, respectively). These results demonstrate that cell proliferation of Nucleus Pulposus and Annulus Fibrosus cells was higher in VIPCC coating compared to coating of mixtures of purified fibronectin and fibrinogen.

### Example 5:

### The growth-stimulation effect of VIPCC and different fibronectin/fibrinogen ratios on cultured Nucleus Pulposus proliferation.

The effect on Nucleus Pulposus cell attachment and proliferation by the different concentrations of fibronectin and fibrinogen mixture was also monitored using the XTT assay. The assay is based on the ability of viable cells to reduce 2,3-Bis(2-methoxy-4-nitro-5- sulfophenyl)-2H-tetrazolium-5-carboxanilide (XTT) from a yellow tetrazolium salt to a soluble orange formazan compound.

Nucleus Pulposus cells were prepared using the same procedure described in Example 1. The cells were dispensed into a flat bottom 96-well plates coated with 30 µl of one of the following solutions: 0.626 µg purified fibronectin, 10 µg purified human fibrinogen, diluted VIPCC (contains 10 µg fibrinogen and 1.57 µg fibronectin) and fibronectin/fibrinogen mixture (0.626 µg: 6.26 µg; 1/10), at a density of 1×10⁴ cells per well. Uncoated wells served as control group.

After 4 h incubation, the growth medium (DMEM/Ham's F12 medium supplemented with the above mentioned antibiotics and 10 % FBS) was replaced to remove unattached cells. The cultures were incubated in a humidified atmosphere of 5% CO₂ and 95% air at 37 °C for 24 hours. At the end of the culture period, 50 µl of XTT reagent (XTT-Cell Proliferation Kit; Biological industries; cat No 20-300-1000) was added to each well, and the cultures were placed in an incubator at 37 °C for 4 hours. The color developed was read by a spectro-ELISA reader according to the manufacture protocol. The supernatant was discarded and fresh growth medium was added. The assay was repeated 5 days later. The absorbance intensity is proportional to the amount of metabolic active cells. As before, results are presented as percentage of fibronectin absorbance intensity (100%).

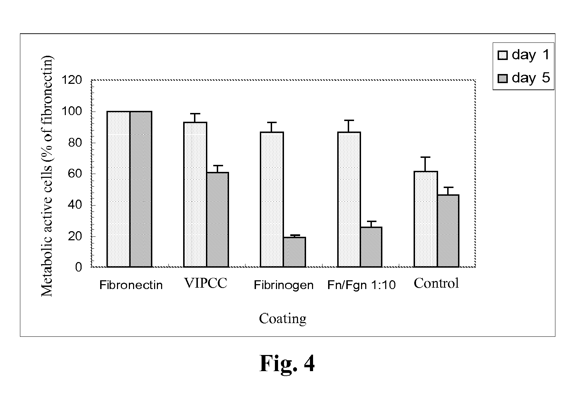

As seen in Fig. 4, fibrinogen coating resulted in a pronounced decrease of Nucleus Pulposus chondrocytes viability (86.4 and 19.6% on day 1 and 5, respectively; Δ=-66.8%). VIPCC, which comprises a fibronectin/fibrinogen ratio in the range of 1/5-1/10, caused a moderate decrease in cell viability compared to fibrinogen/fibronectin-coated plastic culture wells (1/10) (32% decrease compared to 60.9% decrease in VIPCC and fibrinogen/fibronectin mixture, respectively). These results confirm the previous results and show the advantages of using VIPCC as a coating for Nucleus Pulposus cells and/or chondrocytes as a scaffold for cartilage tissue engineering.

### Example 6:

### Effect of fibrinogen and fibronectin levels on Nucleus Pulposus and Annulus Fibrosus cell-attachment

The present study was aimed to determine the effects of fibrinogen and fibronectin on cultured Nucleus Pulposus and Annulus Fibrosus cell attachment. For this purpose, cells derived from the Nucleus Pulposus and Annulus Fibrosus were isolated as described in Example 1. The cell suspension, which contained 1×10⁴ cells per well for both cell types, was placed in a flat bottom 96-well plate on 30 µl fibronectin/fibrinogen-coating. The coating was composed of a constant amount of fibronectin of 0.626 µg and increasing amounts of purified fibrinogen as follows: 0, 3.13, 6.26 (ratio of 1/10 fibronectin), 12.5 and 146 µg. Another set of experiments were done on fibronectin/fibrinogen-coating composed of a constant amount of fibrinogen of 9.125 mg and increasing amounts of purified fibronectin as follows: 0.0313, 0.042, 0.0626 and 0.125 µg.

The culture was incubated for 4 h and the growth medium (DMEM/Ham's F12 medium supplemented with the above mentioned antibiotics and 10 % FBS) was replaced to remove unattached cells. The cultures were incubated for additional 24 hours in a humidified atmosphere of 5% CO₂ and 95% air at 37°C. The effect of fibrinogen on cell attachment was assessed at days 7, 10-13 and 16 by the XTT assay as described above. The absorbance intensity is proportional to the amount of metabolic active cells. The results are presented as percentage of fibronectin absorbance intensity (100%). Data are presented as mean ± S.D. of at least three separated experiments (in each experiment cells were harvested from three different pigs vertebrate).

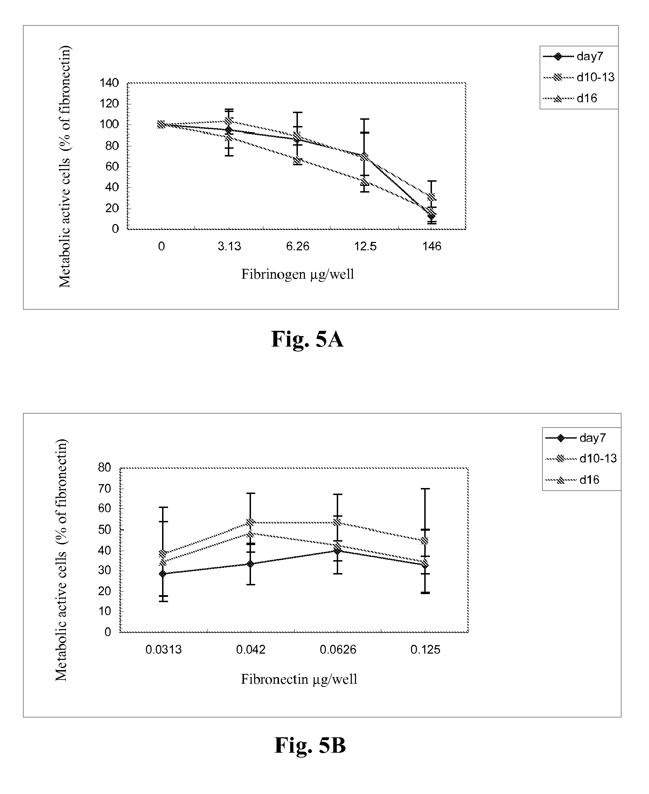

The results are summarized in Fig 5 and show Nucleus Pulposus cell-attachment on constant amount of fibronectin and increased amount fibrinogen in the coating matrix (A) and on constant amount of fibrinogen and increased amount fibronectin in the coating matrix (B). The results show that a constant amount of fibronectin and an increased amount of fibrinogen in the coating lead to a decrease cell attachment of Nucleus Pulposus. Annulus Fibrosus cells were less affected by the fibrinogen addition. Increased amounts of fibronectin in the presence of fixed fibrinogen did not affect cell attachment in both cell types (results of the Annulus Fibrosus chondrocytes cultures are not shown). Additionally, the results are in line with the previous results which indicate that fibronectin/fibrinogen in a ratio of about 1/5 is more suitable for Nucleus Pulposus chondrocytes cell attachment as compared to other fibronectin/fibrinogen ratios.

### Example 7:

### Effect of a three-dimensional scaffold prepared with activated VIPCC on the morphology and function of chondrocytes grown in the scaffold.

Studies (Haudenschild et al. 2001; Li et al. 2006; Peretti et al. 2001) indicate that expansion of chondrocytes in vitro results in phenotype changes, and a decrease proteoglycan synthesis. Therefore, the present experiment was aimed to determine the morphology of chondrocytes cells grown in three-dimensional activated VIPCC. For this purpose, 750 µl of Annulus Fibrosus or Nucleus Pulposus cell suspension was prepared using a 5 fold diluted VIPCC, which contains 14 mg/ml clottable fibrinogen. To activate VIPCC and form a clot, this step was followed by mixing the above mentioned suspension with an equivalent volume of thrombin solution (2 IU, Omrix biopharmaceuticals LTD, IL) into a 6-well plate. A three-dimensional chondrocyte construct, which contained 6 X 10⁵ cells per well was formed.

After formation of the clot the cell-construct was supplemented with 0.5 ml DMEM/Ham's F12 medium containing the above mentioned antibiotics and 10% FBS. The well plate was cultured in 5% CO₂ at 37°C. After 12 hours, the medium was removed and the scaffold was detached from the side wall using a scalpel in order to decreased tension conditions. Undetached cultures, which were considered under more tensed conditions, served as the control group.

The cultures were grown in DMEM/Ham's F12 medium containing the above mentioned antibiotics and 10% FBS and incubated for additional 8 or 14 days, for Annulus Fibrosus or Nucleus Pulposus, respectively. The medium was discarded and the cultures were fixed with 3.7 % paraformaldehyde solution for 10 min, and stained with Alcian blue (Sigma; cat No A5268) for 10 min at room temperature. Alcian blue staining is designed to show Mucopolysaccharides or Glycosaminoglycans (GAGs) characteristically produced by chondrocytes. Chondroitin sulfate, which belongs to the GAGs family, in the form of proteoglycans comprises the ground substance in the extracellular matrix of cartilage tissue.

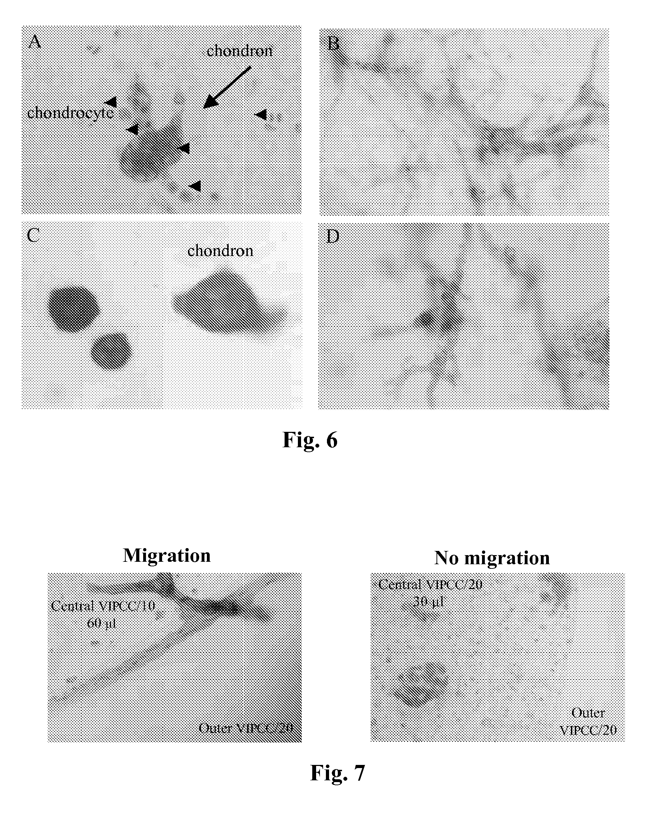

The results are summarized in Fig. 6 and show Alcian blue staining on both Annulus Fibrosus (Fig. 6, upper panel) and Nucleus Pulposus chondrocytes (Fig. 6, lower panel). The results demonstrate that both chondrocytes are functional and able to manufacture chondroitin sulfate. The detached scaffolds (Fig. 6B and D) exhibit filamentous and branched-like phenotype whereas attached and more tensed scaffold (Fig. 6A and C) assumed a sphere-like phenotype. Thus, the results demonstrate that chondrocytes in three-dimensional scaffold grown in activated VIPCC were functional and therefore that VIPCC can be advantageously used as a scaffold for cartilage tissue engineering.

### Example 8:

### Effect of the three-dimensional scaffold's formulation and tension on the chondroconductive characteristic of the VIPCC.

The objective of the present experiment was to investigate the effect of the formulation and tension on the chondro-conductive characteristic of the VIPCC. Isolated Nucleus Pulposus or Annulus Fibrosus chondrocytes suspensions were prepared using three solutions of diluted VIPCC (1:2.5, 1:5, 1:10, which contained a final concentration of 28, 14 and 7 mg/ml clottable fibrinogen, respectively) in DMEM/Ham's F12 medium containing the above mentioned antibiotics. Afterwards, the above mentioned suspension was mixed with an equivalent volume of thrombin component (1 IU/ml, described in US patent 6,121,232). The volume of the final construct was 30, 60 or 90 µl with a final VIPCC dilution of 1:5, 1:10 or 1:20 and of 0.5 IU/ml thrombin. All dilutions contained an equal number of chondrocytes; 1.6X10⁵ cells of Nucleus Pulposus or 1X10⁵ cells of Annulus Fibrosus. The cell suspension was placed in the center of a 24-well plate culture dish. After a clot was formed, 200 µl of a mixture comprised of 0.5 thrombin IU/ml and 20 fold diluted VIPCC, was added at the circumference. After formation of the clot (about 30 min), 0.5 ml growth medium (DMEM/Ham's F12 medium supplemented with antibiotics and 10 % FBS) was added and the plate was incubated for 22 days. The chondro-conductive characteristic of the VIPCC was assessed by evaluating the ability of Nucleus Pulposus or Annulus Fibrosus chondrocytes to transmigrate from the central construct [(comprised of different dilutions of VIPCC and different volumes (30, 60 or 90 µl)] to the peripheral scaffold of VIPCC. After 22 days of culture the medium was discarded and the cultures were fixed with 3.7 % paraformaldehyde solution for 10 min, and stained with Alcian blue (Sigma; cat No A5268) for 10 min at room temperature. By monitoring the localization of the cells in the plate it was found that transmigration occurred exclusively in the 60 µl volumes of 10 fold diluted VIPCC (which contains 7 mg/ml clottable fibrinogen) in both cell types. Fig 7 shows transmigration of Nucleus Pulposus chondrocytes as viewed under the microscope with a magnification of X100 in the 60 µl volume of 10 fold diluted VIPCC (left panel; the cells aligned and formed a belt-like structure) as compared to Nucleus Pulposus chondrocytes in the 30 µl volume of 20 fold diluted VIPCC that were unable to transmigrate (right). Additionally, chondrocytes in all the experimental groups were able to proliferate in the central construct and were capable of producing chondroitin sulfate (results not shown).

### Example 9:

### Biocompatibility of the VIPCC in intervertebral disc injection.

The following experiment was designed to investigate the biocompatibility of Omrix commercial activated-VIPCC in the intervertebral disc. For this purpose, VIPCC and thrombin was injected into the intervertebral disc of a pig.

Female swine (n = 1) weighing 74 kg and at the age of 6 months was housed in an authorized facility according to the current ethical requirements. Anesthesia was induced with an intramuscular mixture of ketamin (10 mg/kg) and xylazine (2 mg/kg).

Physical examination was preformed prior to the surgical procedure (body weight, temperature, heart and respiratory rates). ECG, pulse, and blood pressure were monitored during the procedure.

Surgical procedure: The animal was positioned laterally and bended forward. A contrast agent was used and served as a visual guidance to locate the IVDs cavity using fluorography. The contrast agent used was iodine (Tel Hashomer, IL). The injections were preformed using a syringe connected to 23G spinal needle 90 mm ("phoenix" Kobayashi Shoji K.K Tokyo, Japan).

In order to practice the injection procedure, IVDs T₁₃-T₁₄, T₁₄-T₁₅ and T₁₂-T₁₃, were filled with contrast agent (0.37 g/ml iodine) and IVD T₁₂-T₁₃ were injected with diluted iodine (1:1 with saline).

Once the injection procedure was satisfactory, the sequence of IVD injections was as follows:
1. IVDs L₁-L₂ and L₃-L₄ were injected with ∼ 200-300 µl saline.
2. IVDs L₂-L₃ and L₄-L₅ were injected with VIPCC and thrombin (described in US Patent 7,125,569) applied simultaneously (similar quantity in the total volume of ∼ 200-300 µl) using Omrix injection device to form a solid gel.

Prior to each injection, a very small amount (present at the tip of the needle) of 1:10 diluted contrasting agent was injected to the IVD space in order to verify the exact application site (a small amount was injected so that enough intradiscal space was left for activated-VIPCC injection).

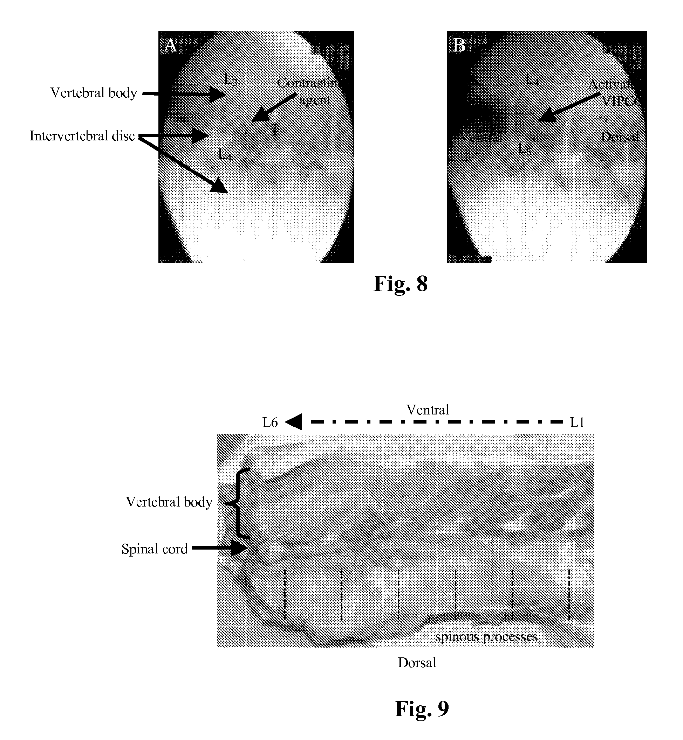

Fig. 8 shows a fluorography image of control (A; saline) and activated-VIPCC (B) injections into the intervertebral disc. The contrast agent turned invisible in the activated-VIPCC injected IVD.

Photographs and digital video recordings were taken after the injection procedure.

Post-operative care: The animal was hospitalized for 14 days and an observation of ambulatory activates (e.g. standing, walking) and drinking, eating and behavior characteristics were monitored. The animal received non-steroidal analgesics (30 mg/kg dipyron) and antibiotics (0.0 2 ml/kg marbocyl) for the first 2 days post operation.

Sample collection: The animal was sacrificed 14 days after surgery and the lumbar spine was removed for histological analysis.

During the observation period the animals drank and ate normally. In the first six days, the animal's ability to wake up was improved. The animal showed weakness of the rear legs and crossed her legs while walking. By the sixth day, the animal was able to walk steadily. No fever was detected during the observation period. On day 13 the animal's weight was 77.5 kg (gained 4.5 kg)

The injected isolated lumber spine showed no sign of damage or inflammation to the surrounding tissue (Fig. 9).

The injected IVDs specimens were isolated, according to the above procedure (Example 1), and decalcified in 8% formic acid until the tissue was soft enough for histological assessment.

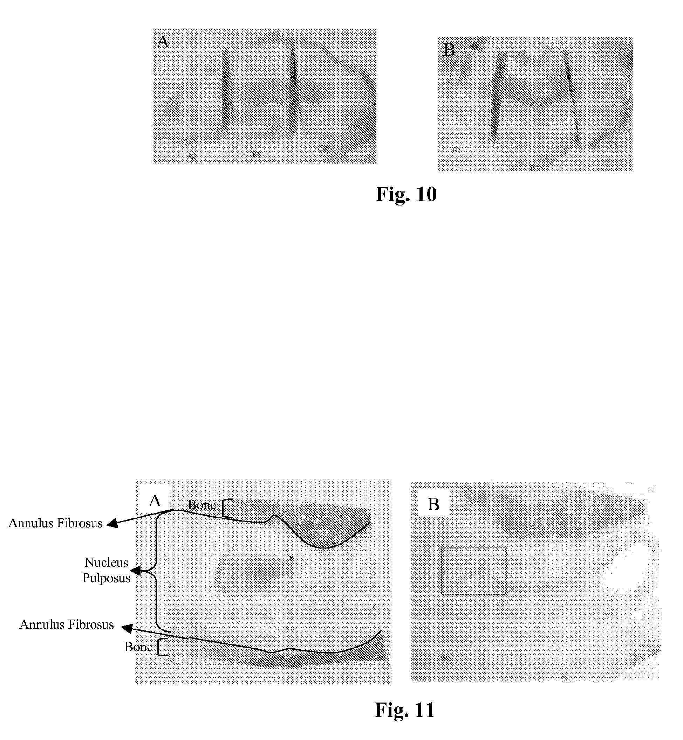

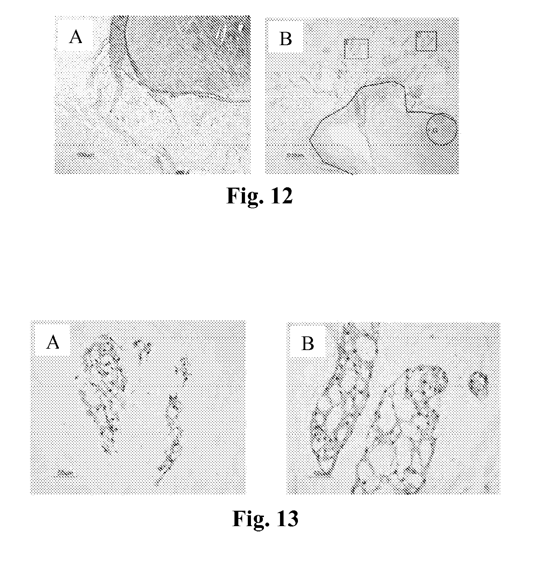

Fig. 10 shows the decalcified IVDs of control and activated-VIPCC injection. Histological assessments were done by Hematoxylin and Eosin staining of the Nucleus Pulposus region of control (A) and activated-VIPCC (B). In IVDs injections (Fig. 11-13), control IVDs (saline injected) showed clusters of chondrocytes with a typical necrotic damage, pyknotic nuclei and karyolysis (Fig. 11A, 13A). Activated-VIPCC injected IVDs showed groups of viable chondrocytes in the peripheral region with a typically vacuolated cytoplasm structure (Fig. 11B, 13B). The central region of the Nucleus Pulposus showed an intense coloring compared to the peripheral region in both experimental groups (Fig. 12A-control and B- activated-VIPCC injections). In the control group the differences were more pronounced. The peripheral region which showed a low intensity color indicates a normal structure with scattered cellular clusters, whereas the intense color indicates necrotic cells. The above shown results indicate that injection with activated-VIPCC prevents structural changes and does not change the typical structure of the intervertebral disc.

### References:

Bar L, Malka O, Naboichenko E, Nur I. The binding of fibrin sealant to collagen is influenced by the method of purification and the cross-linked fibrinogen-fibronectin (heteronectin) content of the 'fibrinogen' component. Blood Coagul Fibrinolysis. 2005;16:111-117.
Benya PD, Shaffer JD. Dedifferentiated chondrocytes reexpress the differentiated collagen phenotype when cultured in agarose gels. Cell. 1982;30:215-224.
Cheng H, Cao Y, Olson L. Spinal cord repair in adult paraplegic rats: partial restoration of hind limb function. Science. 1996;273:510-513.
Gruber HE, Leslie K, Ingram J, Norton HJ, Hanley EN. Cell-based tissue engineering for the intervertebral disc: in vitro studies of human disc cell gene expression and matrix production within selected cell carriers. Spine J. 2004;4:44-55.
Haudenschild DR, McPherson JM, Tubo R, Binette F. Differential expression of multiple genes during articular chondrocyte redifferentiation. Anat Rec. 2001;263:91-98.
Hunter CJ, Matyas JR, Duncan NA. The notochordal cell in the nucleus pulposus: a review in the context of tissue engineering. Tissue Eng. 2003;9:667-677.
Ju YE, Janmey PA, McCormick ME, Sawyer ES, Flanagan LA. Enhanced neurite growth from mammalian neurons in three-dimensional salmon fibrin gels. Biomaterials. 2007; 28: 2097-2108.
Li WJ, Jiang YJ, Tuan RS. Chondrocyte phenotype in engineered fibrous matrix is regulated by fiber size. Tissue Eng. 2006;12:1775-1785.
Miekka SI, Ingham KC, Menache D. Rapid methods for isolation of human plasma fibronectin. Thromb Res. 1982;27:1-14.
Peretti GM, Randolph MA, Zaporojan V, Bonassar LJ, Xu JW, Fellers JC, Yaremchuk MJ. A biomechanical analysis of an engineered cell-scaffold implant for cartilage repair. Ann Plast Surg. 2001;46:533-537.

## Claims

1. A vehicle or construct suitable for delivering a composition of cells comprising notochordal cells or notochordal derived cells into a damaged tissue comprising viral inactivated-plasma activated cryoprecipitate concentrate (VIPCC) having an initial fibronectin/fibrinogen relative concentration higher than 1/12 or about 1/11 to about 1/5 and notochordal cells and/or notochordal derived cells.

2. A vehicle according to claim 1, for delivering cells to intervertebral disc and/or injured central nervous system such as injured spine.

3. The vehicle according to claim 1 or 2, wherein the composition of cells comprises Nucleus Pulposus cells.

4. The vehicle according to claim 3, wherein the composition of cells further comprises Annulus Fibrosus cells.

5. A kit for injecting therapeutic solutions into a damaged spinal intervertebral disc and/or injured central nervous system such as injured spinal cord comprising: a first container comprising viral inactivated-plasma cryoprecipitate concentrate (VIPCC) comprising a fibronectin/fibrinogen ratio of higher than 1/12, or about 1/11 to about 1/5, and a second container comprising an enzyme capable of forming fibrin when it reacts with fibrinogen.

6. The kit according to claim 5, wherein at least one container comprises a contrast agent.

7. The kit according to claim 6, wherein the contrast agent is iodine.

8. A kit comprising: a first container comprising a viral inactivated-plasma cryoprecipitate concentrate (VIPCC) having a fibronectin/fibrinogen ratio of higher than 1/12 or about 1/11 to about 1/5, a second container comprising an enzyme capable of forming fibrin when it reacts with fibrinogen, and a third container comprising a proteolytic enzyme selected from serine peptidases, cystein peptidases, aspartic peptidases, metallo peptidases, hyaluronidase and combinations thereof.

9. The kit according to claim 8, wherein the proteolytic enzyme is selected from trypsin, chymotrypsin, pancreatic elastase, papain chymopapain, pepsin, collagenase, gelatinase, pronase chondroitinase, hyaluronidase and combinations thereof.

10. The kit according to claim 8 or 9, wherein at least one container comprises a contrast agent.

11. The kit according to claim 10, wherein the contrast agent is iodine.

12. A scaffold suitable for repair and/or regeneration of spinal intervertebral disc and/or injured central nervous system such as injured spinal cord when administered in the body comprising viral inactivated-plasma cryoprecipitate concentrate (VIPCC) having an initial fibronectin/fibrinogen relative concentration of higher than1/12 or about 1/11 to about 1/5.

13. The scaffold according to claim 12, wherein the VIPCC is activated VIPCC.

14. A tissue or cell bank comprising notochordal cells and/or notochordal derived cells in a scaffold comprising viral inactivated-plasma cryoprecipitate concentrate (VIPCC) having an initial fibronectin/fibrinogen relative concentration of higher than 1/12 or about 1/11 to about 1/5.

15. The tissue or cell bank according to claim 14, wherein the cells are Nucleus Pulposus cells.

16. A method for enhancing growth, proliferation, differentiation, maintenance, repair and/or restoration of a population of cells comprising cells selected from notochordal cells, notochordal derived cells, cells guided by notocordal cells, comprising contacting the population of said cells with viral inactivated-plasma cryoprecipitate concentrate (VIPCC) having an initial fibronectin/fibrinogen relative concentration of higher than 1/12 or about 1/11 to about 1/5.

17. The method according to claim 16, wherein the VIPCC is activated VIPCC.

18. The method according to claim 16 or 17, wherein said contacting is carried out ex vivo.

19. The method according to claim 16 or 17, wherein said contacting is carried out in vivo.

20. The method according to any one of claims 16 to 19, wherein the population comprises Nucleus Pulposus cells.

21. The method according to claim 20, wherein the population further comprises Annulus Fibrosus cells.

22. A method for treating an intervertebral disc and/or central nervous system disease, disorder or condition comprising administering to a subject in need a kit according to any one of claims 5 to 11, a scaffold according to claim 12 or 13, a tissue or cell bank according to claim 14 or 15 and/or a vehicle according to claim any one of claims 1 to 4.

23. A method for treating an intervertebral disc and/or central nervous system disease, disorder or condition comprising injecting into the disc of a subject in need a viral inactivated-plasma cryoprecipitate concentrate (VIPCC), optionally in combination with a cell composition comprising cells selected from notochordal cells, notochordal derived cells, and cells guided by notocordal cells, wherein the cryoprecipitate comprises an initial fibronectin/fibrinogen relative concentration of higher than 1/12 or a about 1/11 to about 1/5.

24. The method according to claim 23, wherein the VIPCC is activated VIPCC.

25. The method according to claim 23 or 24, wherein the VIPCC comprises an initial fibronectin/fibrinogen relative concentration of about 1/10 to about 1/5.

26. The method according to any one of claims 23 to 25, wherein the VIPCC comprises a contrast agent.

27. The method according to claim 26, wherein the contrast agent is iodine.

28. The method according to any one of claims 23 to 27, for preventing intervertebral disc herniation.

29. The method according to any one of claim 23 to 27, wherein the subject in need is at early stage of intervertebral degenerative disease.

30. The method according to any one of claims 24 to 27, wherein the activated VIPCC serves as a scaffold for reconstruction of Nucleus Pulposus in a subject at advanced stage of intervertebral degenerative disc disease.

31. The method according to any one of claims 24 to 27, wherein the activated VIPCC serves as a scaffold for reconstruction of injured or ruptured spinal cord.

32. The method according to any one of claims 23 to 31 comprising injecting the VIPCC in combination with a cell composition comprising cells selected from notochordal cells, notochordal derived cells, and cells guided by notocordal cells.

33. The method according to claim 32 comprising excising all or a portion of the Nucleus Pulposus from the intervertebral disc prior to injecting the VIPCC and the cells.

34. The method according to claim 32 or 33, wherein the notochordal cells and/or notochordal derived cells are from Nucleus Pulposus tissue excised from the intervertebral disc.

35. The method according to claim 33 or 34, wherein prior to the injection the cells were cultured ex-vivo on VIPCC comprising a fibronectin/fibrinogen ratio of about 1/11 to about 1/5.

36. Use of a kit according to claim 5 to 11, a scaffold according to claim 12 or 13, a tissue or cell bank according to claim 14 or 15 and/or a vehicle according to claim any one of claims 1 to 4, in the manufacture of a medicament for treating intervertebral degenerative disc and/or central nervous system injury such as spine injury.

37. Use of viral inactivated-plasma activated cryoprecipitate concentrate, wherein said cryoprecipitate comprises an initial fibronectin/fibrinogen relative concentration of higher than 1/12 or about 1/11 to about 1/5 for the manufacturing of a medicament for treating an intervertebral degenerative disc, and central nervous system disease, disorder or condition.
